# EUROPEAN PATENT APPLICATION

(11) **EP 2 433 650 A2**
(43) Date of publication of application: **28.03.2012**
(21) Application number: 11190677.2
(22) Date of filing: 05.06.2007
(51) Int. Cl.: A61K 39/395, A61P 17/06, A61P 3/10, A61P 37/06, C07K 16/28

(54) **Administration of anti-CD3 antibodies in the treatment of autoimmune diseases**

(30) Priority: 06.06.2006 US 447628; 06.06.2006 US 921485 P
(62) Divisional of application: 07795754.6
(71) Applicant: Tolerrx Inc., Cambridge, MA 02139 (US)
(72) Inventor: Ponath, Paul, San Francisco, California 94114 (US); Rosenzweig, Michael, Boston, Massachusetts 02116 (US); Vaickus, Louis, Hingham, Massachusetts 02043 (US)
(74) Representative: LOUIS, PÖHLAU, LOHRENTZ

(57) **Abstract**

A method of treating an autoimmune disease, such as, diabetes or psoriasis, by administering an anti-CD3 antibody, or anti-CD3 antibody fragment. The anti-CD3 antibody, or anti-CD3 antibody fragment, is administered over a course of treatment wherein, during the course of treatment, the anti-CD3 antibody, or anti-CD3 antibody fragment is administered in a total amount which does not exceed 300 µg/kg when administered intravenously or, when administered other than intravenously, is administered in a total amount that does not exceed a total amount bioequivalent to 300µg/kg of intravenous administration thereof.

## Description

This application claims the priority of U.S. Provisional Patent Application Serial No. _______, filed June 6, 2006, which Provisional Patent Application is converted U.S. Utility Patent Application No. 11/447,628 filed on June 6, 2006, which Utility Patent Application was converted to such Provisional Patent Application by *Request Under 37 CFR 1,53(c)(2),* filed April 16, 2007, the disclosures of which application(s) are hereby incorporated by reference in their entireties.

This invention relates to the administration of anti-CD3 antibodies to treat an autoimmune disease, such as diabetes or psoriasis. In one embodiment, this invention relates to the administration of anti-CD3 antibodies or fragments thereof, to treat an autoimmune disease, such as diabetes or psoriasis, at reduced dosage levels.

Anti-CD3 antibodies, or fragments thereof, have been employed in the treatment of autoimmune diseases, including diabetes. For example, U.S. Patent No. 7,041,289 and published Canadian Patent Application No. 2,224,256 teach the treatment of autoimmune diseases, including diabetes, by administering an anti-CD3 antibody, or fragment thereof, in an amount of 5 to 20 mg per dose. In one embodiment, the anti-CD3 antibody is administered in an amount of 5 to 10 mg/day for 10 to 14 days.

Herold, et al., disclose a clinical study in which patients with Type I diabetes were given a monoclonal anti-CD3 antibody, hOKT3γl (Ala-Ala). In the study, the patients were given a total dose of 500 µg/kg of the antibody over a period of 14 days. The dosages given over the 14-day course of treatment were as follows:
Day 1 - 1.42 µg/kg
Day 2 - 5.67 µg/kg
Day 3 - 11.3 µg/kg
Day 4 - 22.6 µg/kg
Day 5-14 - 45.4 µg/kg

After the first cohort of subjects were treated, the total dose was increased to 620 µg/kg. (Herold, et al., Immunologic Research, Vol. 28, No. 2, pgs. 141-150 (2003); Herold, et al., New Engl. J. Med., Vol. 346, No. 22, pgs. 1692-1698 (May 30, 2002); Herold, et al., J. Clin. Invest., Vol. 111, No. 3, pgs. 409-418 (February 2003); Herold, et al., J. Clin. Invest., Vol. 113, No. 3, pgs. 346-349 (February 2004); Herold, et al., Diabetes, Vol. 54, pgs. 1763-1769 (June 2005).) Peak serum levels of 0.25 µg/ml were obtained, during days 5 to 14 of the treatment. (Herold, et al., 2002; Herold, et al., Immunologic Research, 2003.)

In accordance with an aspect of the present invention, there is provided a method of treating an autoimmune disease in an animal. In one embodiment, the disease is an established, spontaneous, and ongoing autoimmune disease. The method comprises administering to an animal an anti-CD3 antibody or anti-CD3 antibody fragment selected from the group consisting of anti-CD3 antibodies and fragments thereof which do not bind or have reduced binding to the Fc (gamma) receptors. The anti-CD3 antibody, or anti-CD3 antibody fragment thereof, is administered in a therapeutically effective amount over a course of treatment wherein during the course of treatment, the total amount of anti-CD3 antibody or anti-CD3 antibody fragment, when intravenously administered is administered in an amount which does not exceed 300 µg/kg.

The terms "anti-CD3 antibody" and "anti-CD3 antibody fragment", as used herein, mean antibodies or antibody fragments which recognize or bind to CD3.

As is known in the art, the term "µg/kg" means the amount of antibody and/or fragment thereof delivered, in micrograms, per kilogram of body weight of the animal being treated.

In one non-limiting embodiment, the autoimmune disease is diabetes. In another non-limiting embodiment, the autoimmune disease is psoriasis.

If administered other than intravenously, then such anti-CD3 antibody or anti-CD3 antibody fragment is administered in an amount such that the resulting area under the curve (AUC) from such administration is from about 80% to about 120% of the AUC produced by intravenous administration of such anti-CD3 antibody or anti-CD3 antibody fragment. Thereafter, such amounts are referred to as being an amount bioequivalent to intravenous administration.

The terms "area under the curve" and "AUC" are terms well known in the pharmaceutical arts. An AUC value is calculated by plotting a graph with data from systemic concentration of a therapeutic agent as a function of time, with the X-axis generally representing time and the Y-axis generally representing concentration of therapeutic agent. The area under the line formed by joining the various data points then is integrated into a numerical value. AUC values for the anti-CD3 antibody or anti-CD3 antibody fragment may be subject to inter-and intra-patient variation due to physiological and/or environmental factors present in individual patients during the administration of the anti-CD3 antibodies or anti-CD3 antibody fragments, in various formulations and/or compositions.

In one non-limiting embodiment, the anti-CD3 antibody, or anti-CD3 antibody fragment, is administered intravenously over a course of treatment in which the total amount of anti-CD3 antibody or anti-CD3 antibody fragment administered during the course of treatment does not exceed 175 µg/kg, and in another embodiment, such amount does not exceed 150 µg/kg, and in some embodiments, such amount does not exceed 50 µg/kg, or in the case of administration other than intravenous, in an amount bioequivalent to intravenous administration in such amounts.

In another non-limiting embodiment, the anti-CD3 antibody or anti-CD3 antibody fragment is administered intravenously over a course of treatment wherein the total amount of anti-CD3 antibody or anti-CD3 antibody fragment administered during the course of treatment is at least 8 µg/kg or in the case of administration other than intravenous, in an amount bioequivalent to intravenous administration in such amounts.

In another embodiment, the anti-CD3 antibody or anti-CD3 antibody fragment, on each day of treatment is administered intravenously in an amount which generally is not less than 0.1 µg/kg per day, or in the case of administration other than intravenously, in an amount bioequivalent to intravenous administration in such amount.

Over the course of treatment, the same dosage of anti-CD3 antibody or anti-CD3 antibody fragment may be given each day over the course of treatment, or different doses of the anti-CD3 antibody or anti-CD3 antibody fragment may be given on each day of the course of treatment. For example, the dose of anti-CD3 antibody or anti-CD3 antibody fragment may be varied on each day of the course of treatment, provided the total dosage of anti-CD3 antibody or anti-CD3 antibody fragment does not exceed 300 µg/kg. In one embodiment, the amount of anti-CD3 antibody or anti-CD3 antibody fragment given intravenously on any one day of the course of treatment does not exceed 30 µg/kg/day. In another embodiment, the amount does not exceed 25 µg/kg/day. In yet another embodiment, the amount does not exceed 20 µg/kg/day. In still another embodiment, the amount does not exceed 15 µg/kg/day, and in yet another embodiment the amount does not exceed 10 µg/kg/day or, in the case of administration other than intravenous, in an amount which does not exceed an amount bioequivalent to intravenous administration in such amounts.

In one embodiment, the course of treatment with respect to the various embodiments hereinabove described does not exceed 10 days. In another embodiment, the course of treatment does not exceed 8 days. In yet another embodiment, the course of treatment does not exceed 6 days. In a further embodiment, the course of treatment does not exceed 4 days. In another embodiment, the course of treatment does not exceed 3 days. In another embodiment, the course of treatment does not exceed one day.

In one embodiment, the dose of anti-CD3 antibody or anti-CD3 antibody fragment which is administered is increased on each succeeding day of the treatment, with the greatest dosage of anti-CD3 antibody or anti-CD3 antibody fragment being administered on the last day of the treatment. In another embodiment, during the course of the treatment, an initial dose of anti-CD3 antibody or anti-CD3 antibody fragment is administered on the first day of the treatment. The dose then is increased on each succeeding day of the treatment until a preselected maximum daily dosage is reached. Then such maximum daily dosage is administered on each succeeding day of the remaining days of the treatment. In each case, the total dosage over the course of treatment does not exceed 300 µg/kg.

In one embodiment, the anti-CD3 antibody or anti-CD3 antibody fragment is administered in an amount of about 4.3 µg/kg on Day 1 of the treatment, and in an amount of about 7.1 µg/kg on Day 2 of the treatment. From Day 3 to Day 8 or 9 of the treatment, the anti-CD3 antibody or anti-CD3 antibody fragment is administered in an amount of about 14.3 µg/kg on each of such days of the treatment.

In another embodiment, the anti-CD3 antibody or anti-CD3 antibody fragment is administered in an amount of about 1.4 µg/kg on Day 1 of the treatment, in an amount of about 2.8 µg/kg on Day 2 of the treatment, and in an amount of about 4.3 µg/kg on Day 3 of the treatment. From Day 4 to Day 8 of the treatment, the anti-CD3 antibody or anti-CD3 antibody fragment is administered in an amount of about 7.1 µg/kg on each of such days of the treatment.

In a further embodiment, the anti-CD3 antibody or anti-CD3 antibody fragment is administered in an amount of about 1.4µg/kg on Day 1 of the treatment, in an amount of about 2.8µg/kg on Day 2 of the treatment, and in an amount of about 4.3µg/kg on Day 3 of the treatment. From Day 4 to Day 8 of the treatment, the anti-CD3 antibody or anti-CD3 antibody fragment is administered in an amount of about 10.7µg/kg on each of such days of the treatment.

In yet another embodiment, the anti-CD3 antibody or anti-CD3 antibody fragment is administered in an amount of about 1.4µg/kg on Day 1 of the treatment, in an amount of about 2.8µg/kg on Day 2 of the treatment, and in an amount of about 4.3µg/kg on Day 3 of the treatment. From Day 4 to Day 8 of the treatment, the anti-CD3 antibody or anti-CD3 antibody fragment is administered in an amount of about 14.3µg/kg on each of such days of the treatment.

The anti-CD3 antibody, or anti-CD3 antibody fragment, may be administered systemically, such as by intravenous, intra-arterial, intraperitoneal, intramuscular, or subcutaneous administration. In general, when the anti-CD3 antibody or anti-CD3 antibody fragment is administered by methods other than by intravenous administration, such as by intramuscular or subcutaneous administration, the area under the curve (AUC) resulting from such administration is from about 80% to 120% of the area under the curve resulting from intravenous administration. Thus, when administered by methods other than by intravenous administration, such as by intramuscular or subcutaneous administration, the anti-CD3 antibody, or anti-CD3 antibody fragment, may be administered in doses higher or lower than the hereinabove described maximum dose for intravenous administration, provided that such amount administered other than intravenously does not exceed an amount bioequivalent to that of a dose that is bioequivalent to the maximum intravenous dose.

The anti-CD3 antibody or anti-CD3 antibody fragment may be a human antibody, an animal antibody, such as a non-human mammalian antibody, such as a rodent antibody, including but not limited to mouse and rat antibodies, a chimeric antibody, or a humanized antibody. Alternatively, the anti-CD3 antibody or anti-CD3 antibody fragment may include a combination of human, animal, chimeric, and/or humanized portions.

The anti-CD3 antibody or anti-CD3 antibody fragment may be a monoclonal or polyclonal antibody or fragment thereof. The anti-CD3 antibody, or anti-CD3 antibody fragment, in one embodiment, is a monoclonal antibody or antibody fragment, such as an F (ab')₂ fragment.

In another embodiment, the anti-CD3 antibody, or anti-CD3 antibody fragment, has an Fc region which is removed or modified, whereby binding of the anti-CD3 antibody or anti-CD3 antibody fragment to the Fc (gamma) receptors is reduced or eliminated. In one embodiment, the Fc region is aglycosylated, whereby binding to the Fc (gamma) receptors is reduced or eliminated.

For example, human Fc regions of IgG antibodies are known to be glycosylated at the asparagine residue at position 297, which makes up part of the N-glycosylation motif Asn²⁹⁷-X²⁹⁸-Ser²⁹⁹ or Thr²⁹⁹, wherein X is any amino acid residue except proline. The anti-CD3 antibody may be aglycosylated by the replacement of Asn²⁹⁷ in the Fc region with another amino acid which cannot be glycosylated. Any other amino acid may be used. In one embodiment, Asn²⁹⁷ is replaced with Ala²⁹⁷. Alternatively, glycosylation at Asn²⁹⁷ can be prevented by altering one of the other amino acid residues of the motif, such as, for example, by replacing amino acid residue 298 with proline, or amino acid residue 299 with any amino acid other than serine or threonine. Techniques for effecting such aglycosylation are well known to those skilled in the art, such as, for example, by site-directed mutagenesis. Examples of monoclonal anti-CD3 antibodies which are aglycosylated at amino acid residue 297 of the Fc region are described in U.S. Patent Nos. 5,585,097; 5,968,509; and 6,706,265, the contents of which are incorporated herein by reference.

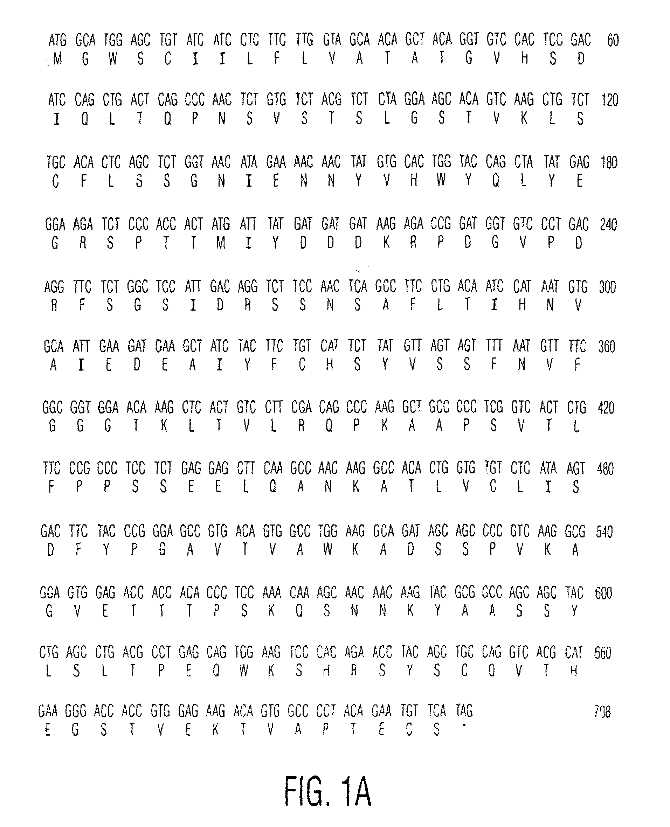

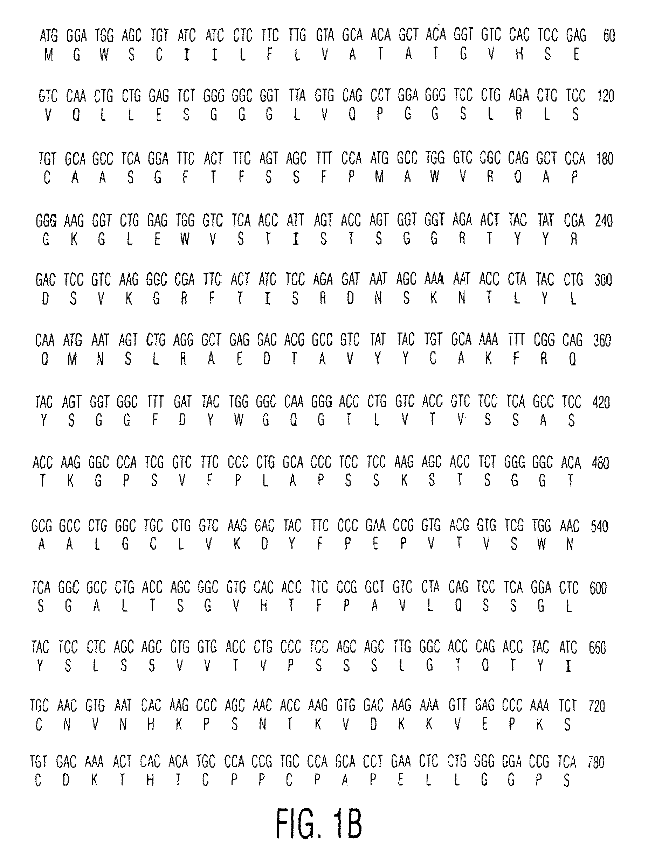

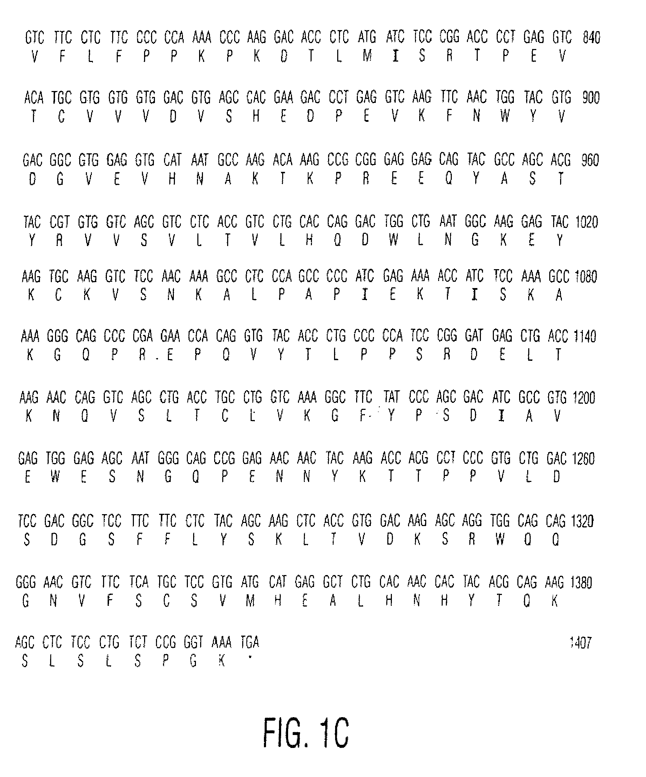

In one embodiment, the anti-CD3 antibody includes a humanized heavy chain in which, the complementarity determining regions, or CDRs, of the variable region of the heavy chain are rat CDRs and the remainder of the heavy chain is human, and a chimeric light chain in which the variable region is a rat variable region and the constant region is a human constant region, except that amino acid residues 1, 2, 3, 4, and 7 of the rat variable region have been mutated. In addition, the asparagine residue at position 297 of the Fc region has been replaced with alanine, whereby the Fc region has become aglycosylated. Such antibody sometimes is hereinafter referred to as "TRX4 antibody." The amino acid sequences of the light and heavy chains of and the nucleotide sequences encoding the light and heavy chains of the TRX4 antibody are shown in Figure 1.

In another embodiment, the Fc region is glycosylated; however, amino acid residues other than Asn²⁹⁷ of the Fc region have been deleted and/or mutated such that binding of the anti-CD3 antibody or fragment to the Fc (gamma) receptors has been reduced or eliminated.

For example, in one embodiment, each of the amino acid residues Leu²³⁴ and Leo²³⁵ of the Fc region have been changed to Ala²³⁴ and Ala²³⁵, whereby binding to the Fc (gamma) receptors is eliminated. An example of such an antibody is the hOKT3γl (Ala-Ala) antibody, which is a humanized anti-CD3 monoclonal antibody that contains the binding region of the murine monoclonal antibody OKT3 (U.S. Patent No. 4,658,019) on a human IgG1, and wherein amino acid residues Leu²³⁴ Leu²³⁵ are replaced with alanine residues. Such antibody is described further in Herold, et al., Immunologic Research, Vol. 28, No. 2, pgs. 141-150 (2003), and Xu, et al., Cell Immunol., Vol. 200, No. 1, pgs. 16-26 (2000). Such modifications may be used in a humanized or chimeric antibody.

In another embodiment, the anti-CD3 antibody or anti-CD3 antibody fragment is of an isotype which does not bind to the Fc (gamma) receptors, such as IgA or IgD.

In one embodiment, the anti-CD3 antibodies or anti-CD3 antibody fragments employed in accordance with the present invention have a mitogenicity in humans which has been reduced or eliminated, i.e., such anti-CD3 antibodies or anti-CD3 antibody fragments do not induce mitosis or have a reduced ability to induce mitosis in the presence of human serum. The determination of whether an anti-CD3 antibody or anti-CD3 antibody fragment is non-mitogenic or has reduced mitogenicity may be made by techniques known to those skilled in the art, such as by testing such anti-CD3 antibodies or anti-CD3 antibody fragments in human serum in vitro.

The anti-CD3 antibody, or anti-CD3 antibody fragment, is administered to an animal in the amounts hereinabove described in order to treat an autoimmune disease, such as diabetes or psoriasis, in the animal. The animal may be a mammal, including human and non-human primates.

The anti-CD3 antibody, or anti-CD3 antibody fragment, may be administered in conjunction with an acceptable pharmaceutical carrier or diluent. Suitable pharmaceutical carriers or diluents include, but are not limited to, saline, dextrose, Ringer's lactate solution, or combinations thereof, water, or any other physiological solution used for intravenous administration. The selection of an appropriate pharmaceutical carrier or diluent is within the scope of those skilled in the art.

In another embodiment, the anti-CD3 antibody or anti-CD3 antibody fragment is lyophilized. In such embodiment, the lyophilized anti-CD3 antibody or anti-CD3 antibody fragment is admixed with a carrier or diluent such as those hereinabove described at the time of administration.

In yet another embodiment, the anti-CD3 antibody or anti-CD3 antibody fragment is conjugated to a compound such as a polymer. In one embodiment, the polymer is a polyalkylene glycol. In one embodiment, the polyalkylene glycol is polyethylene glycol, or PEG.

The anti-CD3 antibody, or anti-CD3 antibody fragment, and an acceptable pharmaceutical carrier or diluent may be packaged in an appropriate container, such as a sterile vial. Such vial, in one embodiment, is packaged with instructions to administer the anti-CD3 antibody, or anti-CD3 antibody fragment, over a course of treatment wherein, during the course of treatment, the anti-CD3 antibody, or anti-CD3 antibody fragment, is administered intravenously over the course of treatment wherein during the course of treatment, the total amount of anti-CD3 antibody or anti-CD3 antibody fragment does not exceed 300 µg/kg/day, or with instructions to administer other than intravenously during a course of treatment wherein over the course of treatment, the total amount of anti-CD3 antibody or anti-CD3 antibody fragment administered does not exceed the bioequivalent to intravenous administration of the amounts hereinabove described.

When the anti-CD3 antibody or anti-CD3 antibody fragment is lyophilized, the lyophilized anti-CD3 antibody or anti-CD3 antibody fragment is packaged in a first sterile vial, and the pharmaceutical carrier or diluent is packaged in a second sterile vial. The two vials are packaged with instructions which, in addition to the instructions hereinabove described, also include instructions to add the pharmaceutical carrier or dilulent to the anti-CD3 antibody or anti-CD3 antibody fragment, followed by administration of the anti-CD3 antibody or anti-CD3 antibody fragment and pharmaceutical carrier or diluent to the patient as hereinabove described.

In accordance with another aspect of the present invention, there is provided a method of effecting T-cell receptor modulation in an animal. The method comprises administering to an animal an anti-CD3 antibody or anti-CD3 antibody fragment selected from the group consisting of anti-CD3 antibodies and fragments thereof which do not bind or have reduced binding to the Fc (gamma) receptors. The anti-CD3 antibody, or anti-CD3 antibody fragment thereof, is administered in an amount effective to effect T-cell receptor Modulation in the animal. In one embodiment, the animal is selected from those hereinabove described.

In one embodiment, the anti-CD3 antibody or anti-CD3 antibody fragment is selected from those hereinabove described.

The term "T-cell receptor modulation" as used herein, means a decrease in the number of T-cell receptor (TCR) complex molecules or subunits on the surface of T-cells. In general, a T-cell receptor (TCR) includes alpha and beta (alpha/beta) subunits or gamma and delta (gamma/delta) subunits. The CD3 complex include epsilon, gamma, and delta subunits. The CD3 subunits and zeta subunits form a complex with the subunits of the TCR. The decrease in the number of TCR complex molecules or subunits may result from factors including, but not limited to, the internalization of TCR complex molecules or subunits at a rate which exceeds re-expression of TCR complex molecules or subunits on the surface of T-cells. In one embodiment, the binding of the anti-CD3 antibody or anti-CD3 antibody fragment to the CD3/TCR complex provides for internalization of the alpha/beta or gamma/delta subunits of the TCR, and such internalization is effected at a rate which exceeds re-expression of such TCR alpha/beta or gamma/delta subunits on the surface of T-cells.

In one embodiment, the anti-CD3 antibody or anti-CD3 antibody fragment is administered intravenously in the amounts and/or courses of treatment hereinabove described, or when administered other than intravenously, in amounts bioequivalent to those of intravenous administration.

The anti-CD3 antibody, or anti-CD3 antibody fragment, and an acceptable pharmaceutical carrier (such as those hereinabove described) may be packaged as hereinabove described, with instructions to administer the anti-CD3 antibody or anti-CD3 antibody fragment intravenously in an amount effective to effect TCR modulation, or with instructions to administer other than intravenously in an amount bioequivalent to an effective intravenous amount for TCR modulation.

The invention now will be described with respect to the following drawings, wherein:
Figure 1 shows the amino acid sequences of the light and heavy chains of the TRX4 antibody, and the nucleic acid sequences encoding the light and heavy chains of the TRX4 antibody;
Figure 2 is a graph showing the mean absolute number of CD4+ T-cells in patients before and after such patients were given TRX4 antibody;
Figure 3 is a graph showing the mean absolute number of CD8+ T-cells in patients before and after such patients were given TRX4 antibody;
Figure 4 is a graph showing detection of cell-bound TRX4 antibody with anti-human IgG on CD4+ T -cells in patients before and after such patients were given TRX4 antibody;
Figure 5 is a graph showing modulation of T-cell receptor (TCR) αβ sites in patients as detected by staining CD4+ T-cells, before and after such patients were given TRX4 antibody, with a non-competing T-cell receptor antibody;
Figure 6 is a graph showing free CD3 sites on CD4+ T -cells in patients, before and after such patients were given TRX4 antibody, as detected with biotinylated TRX4;
Figure 7 is a graph showing the mean amount of (± standard deviation) amount of TRX4 antibody bound to CD4+ T cells in a cohort of patients who received 0.1 mg TRX4 on three successive days;
Figure 8 is a graph showing the mean (± standard deviation) absolute number of lymphocytes in four cohorts of patients who received TRX4. The first cohort received 0.1 mg TRX4 on three successive days. The second cohort received 0.5 mg TRX4 on three successive days. The third cohort received 0.1 mg TRX4 on Day 1, 0.3 mg TRX4 on Day 2, and 0.5 mg TRX4 on Day 3. The fourth cohort received one injection of 0.3 mg TRX4;
Figure 9 is a graph showing the mean (± standard deviation) absolute number of CD4+ lymphocytes in the four cohorts mentioned with respect to Figure 8 hereinabove;
Figure 10 is a graph showing the mean (± standard deviation) absolute number of CD8+ lymphocytes in the four cohorts mentioned with respect to Figure 8 hereinabove;
Figure 11 is a graph showing the mean (± standard deviation) absolute number of T-cell receptor positive (TCR+) T lymphocytes (CD4+ and CD8+) in the four cohorts mentioned with respect to Figure 8 hereinabove;
Figure 12 is a graph showing the mean (± standard deviation) absolute number of CD2+ T lymphocytes (CD4+ and CD8+) in the four cohorts mentioned with respect to Figure 8 hereinabove;
Figure 13 is a graph showing the mean (± standard deviation) absolute number of CD4+ T cells pre-and post-TRX4 dosing in a cohort of patients who received 0.1 mg TRX4 on Day 1, 0.2 mg TRX4 on Day 2, 0.3 mg TRX4 on Day 3, and 0.5 mg TRX4 on Day 4;
Figure 14 is a graph showing the mean (± standard deviation) amount of TRX4 antibody bound to CD4+ T cells in the cohort mentioned with respect to Figure 13 hereinabove;
Figure 15 is a graph showing the modulation of TCR αβ sites measured in MESF units (± standard deviation) as detected by staining CD4+ T-cells pre- and post- TRX4 dosing, with a non-competing TCR antibody; in the cohort mentioned with respect to Figure 13 hereinabove;
Figure 16 is a graph showing the number (± standard deviation), measured in MESF units, of free CD3 sites, on CD4+ T cells pre- and post- TRX4 dosing, as detected with biotinylated TRX4, in the cohort mentioned with respect to Figure 13 hereinabove;
Figure 17 is a graph showing the modulation of TCR, αβ sites, measured in MESF units (± standard deviation), as detected by staining CD4+ T-cells pre- and post- TRX4 dosing with a non-competing TCR antibody in a cohort of patients that received 0.1 mg TRX4 on Day 1, 0.2 mg TRX4 on Day 2, 0.3 mg TRX4 on Day 3, and 0.75 mg TRX4 on Day 4;
Figure 18 is a graph showing the number (± standard deviation), measured in MESF units, of free CD3 sites on CD4+ T-cells, as detected with biotinylated TRX4, in the cohort mentioned with respect to Figure 17 hereinabove;

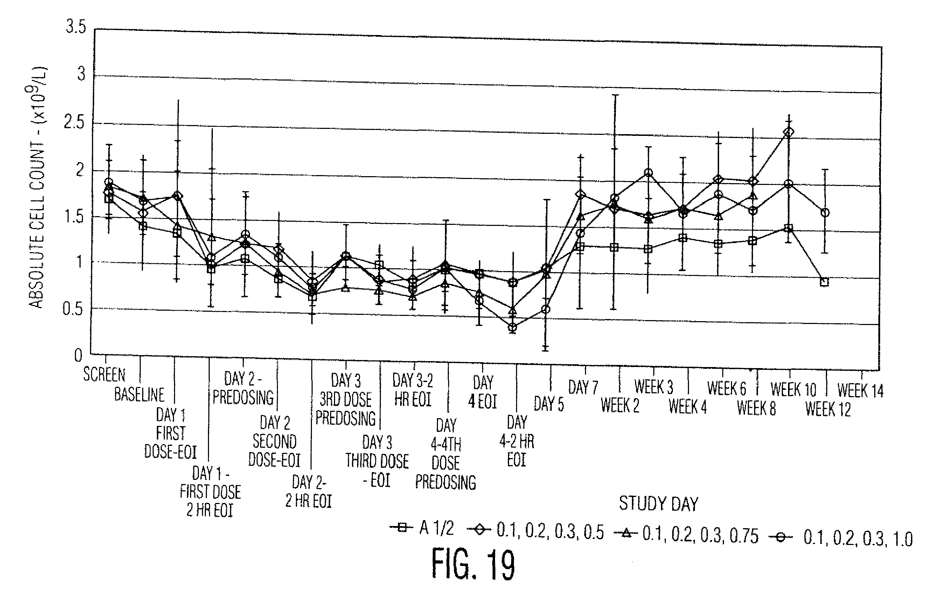

Figure 19 is a graph showing the mean (± standard deviation) absolute numbers of lymphocytes in four cohorts of patients who received TRX4. The first cohort (A ½) received 0.05 mg TRX4 on Day 1, 0.1 mg TRX4 on Day 2, 0.15 mg TRX4 on Day 3, and 0.25 mg TRX4 on Day 4. The second cohort received 0.1 mg TRX4 on Day 1, 0.2 mg TRX4 on Day 2, 0.3 TRX4 on Day 3, and 0.5 TRX4 on Day 4. The third cohort received 0.1 mg TRX4 on Day 1, 0.2 mg TRX4 on Day 2, 0.3 mg TRX4 on Day 3, and 0.75 TRX4 on Day 4. The fourth cohort received 0.1 mg TRX4 on Day 1, 0.2 mg TRX4 on Day 2, 0.3 mg TRX4 on Day 3, and 1.0 mg TRX4 on Day 4;
Figure 20 is a graph showing the mean (± standard deviation) absolute number of CD4+ lymphocytes in the four cohorts mentioned with respect to Figure 19 hereinabove;
Figure 21 is a graph showing the mean (± standard deviation) absolute number of CD8+ lymphocytes in the four cohorts mentioned with respect to Figure 19 hereinabove;
Figure 22 is a graph showing the mean (± standard deviation) absolute number of TCR+ T lymphocytes (CD4+ and CD8+) in the four cohorts mentioned with respect to Figure 19 hereinabove;
Figure 23 is a graph showing the mean (± standard deviation) absolute number of CD2+ T lymphocytes (CD4+ and CD8+) in the four cohorts mentioned with respect to Figure 19 hereinabove;
Figure 24 is a graph showing the mean (± standard deviation) amount, measured in MESF units, of TRX4 antibody bound to CD4+ T-cells, as detected by an anti-human IgG antibody on CD4+ T-cells in a cohort of patients who received 0.1 mg TRX4 on Day 1, 0.2 mg TRX4 on Day 2, 0.3 mg TRX4 on Day 3, and 0.5 mg TRX4 on each of Days 4 through 8, in a protocol entitled TRX4 Therapeutic Evaluation of Different Multi-Dose Regimens in Type 1 Diabetes Mellitus, or TTEDD;
Figure 25 is a graph showing the mean (± standard deviation) amount, measured in MESF units, of TRX4 antibody bound to CD8+ T-cells, as detected by an anti-human IgG antibody on CD8+ T-cells, in the cohort of patients mentioned with respect to Figure 24 hereinabove;
Figure 26 is a graph showing the number (± standard deviation), measured in MESF units, of free CD3 sites on CD4+ T-cells, as detected with biotinylated TRX4, in the cohort mentioned with respect to Figure 24 hereinabove;
Figure 27 is a graph showing the number (± standard deviation), measured in MESF units, of free CD3 sites on CD8+ T-cells, as detected with biotinylated TRX4, in the cohort mentioned with respect to Figure 24 hereinabove;
Figure 28 is a graph showing the number (± standard deviation) of TCR sites, expressed as percent of baseline, detected on CD4+ T-cells with a non-competing antibody, in the cohort mentioned with respect to Figure 24 hereinabove;
Figure 29 is a graph showing the number (± standard deviation) of TCR sites, expressed as percent of baseline, detected on CD8+ T-cells with a non-competing antibody, in the cohort mentioned with respect to Figure 24 hereinabove;
Figure 30 is a graph showing the mean (± standard deviation) absolute number of CD4+ T-cells in the cohort of patients mentioned with respect to Figure 24 hereinabove;
Figure 31 is a graph showing the mean (± standard deviation) absolute number af CD8+ T-cells in the cohort of patients mentioned with respect to Figure 24 hereinabove;
Figure 32 is a graph showing the mean (± standard deviation) absolute number of CD19+ B-cells in the cohort of patients mentioned with respect to Figure 24 hereinabove;
Figure 33 is a graph showing the mean (± standard deviation) absolute number of lymphocytes in four cohorts of patients who received an anti-CD3 antibody or a placebo. The first cohort (EU) received 8 mg of the anti-CD3 antibody TRX4 each day for six consecutive days in a Phase II trial in Europe. The second cohort received a placebo. The third cohort (TTEDD) received 0.1 mg TRX4 on Day 1, 0.2 mg TRX4 on Day 2, 0.3 mg TRX4 on Day 3, and 0.5 mg TRX4 on each of Days 4 through 8. The fourth cohort (PSO) was a cohort of psoriasis patients that received a single intravenous infusion of 1 mg TRX4;
Figure 34 is a graph showing the mean (± standard deviation) absolute number of CD2+ T lymphocytes in the first, second, and third cohorts mentioned with respect to Figure 33 hereinabove;
Figure 35 is a graph showing the mean (± standard deviation) absolute number of CD8+ T lymphocytes in the four cohorts mentioned with respect to Figure 33 hereinabove;
Figure 36 is a graph showing the mean (± standard deviation) absolute number of CD4+ T lymphocytes in the four cohorts mentioned with respect to Figure 33 hereinabove;
Figure 37 is a graph showing the mean (± standard deviation) absolute number of TCR+ T lymphocytes (CD4+ and CD8+) in the four cohorts mentioned with respect to Figure 33 hereinabove; and
Figure 38 is a graph showing the mean cytokine release of the cytokines TNF-α and IL-6 in the first (EU) and third (TTEDD) cohorts mentioned with respect to Figure 33 hereinabove.

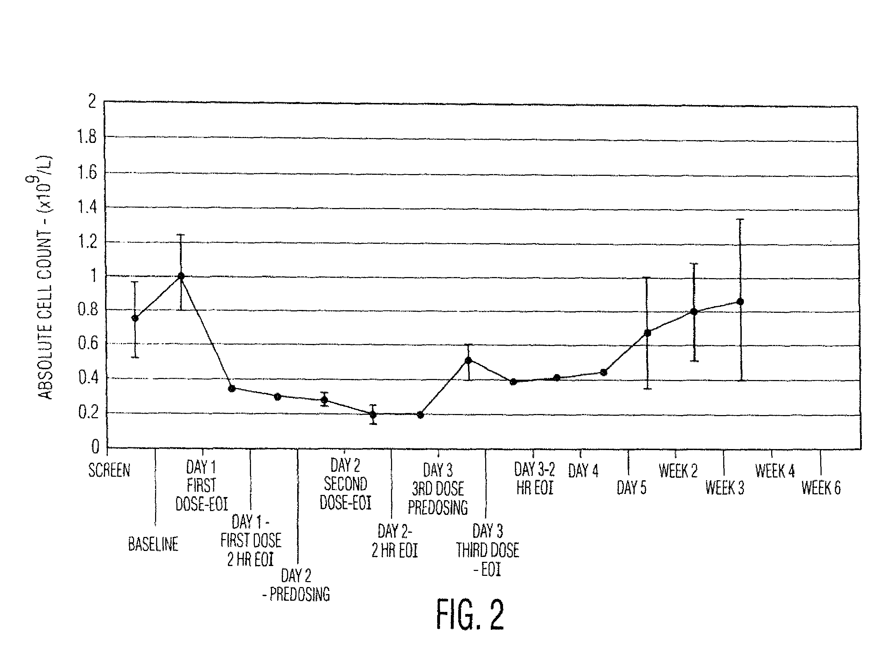

In each of Figures 2 through 38, the term "EOI" used therein means "end of infusion."

### EXAMPLES

The invention now will be described with respect to the following examples; however, it is to be understood that the scope of the present invention is not intended to be limited thereby.

In the following examples, when dosages are given in terms of µg/kg, such dosages were calculated based on the average weight of a patient as being 70 kg.

### EXAMPLE I

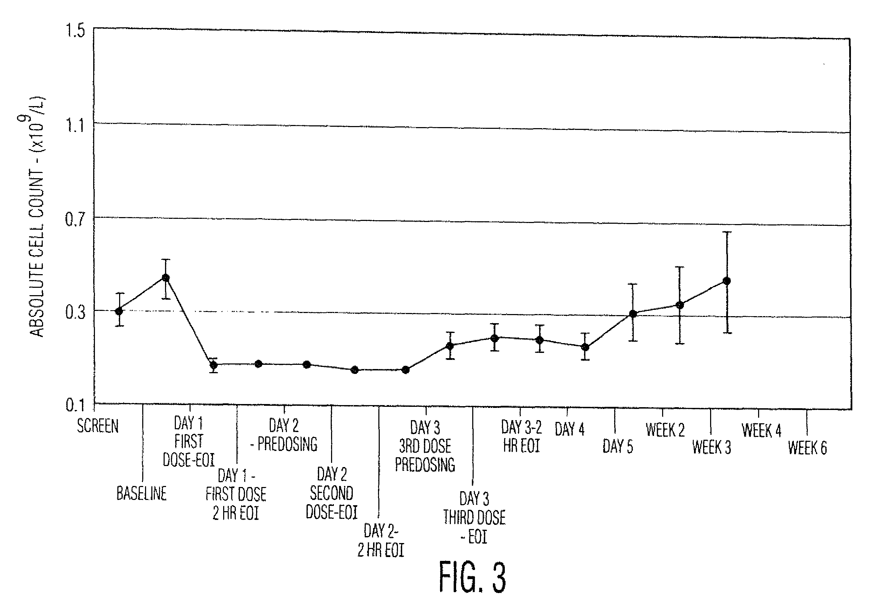

TRX4 antibody, which is an anti-CD3 antibody having a humanized heavy chain and a chimeric light chain, and has an aglycosylated Fc region, in which Asn²⁹⁷ has been mutated to Ala²⁹⁷, was administered intravenously at a dose of 0.5mg, or about 7.1µg/kg, for three consecutive days to each of three patients diagnosed with Type I diabetes. The amino acid sequences of the light and heavy chains, and the nucleic acid sequences encoding such light and heavy chains of TRX4 are shown in Figure 1. CD4+ and CD8+ T-lymphocytes were counted before, during, and after dosing. The absolute number of lymphocytes was determined by multiplying the total white cell count from the complete blood count by the percentage of lymphocytes as determined by a differential cell determination by flow cytometry. Absolute counts for each of CD4+ and CD8+ T-lymphocytes were calculated by multiplying the absolute number of lymphocytes in the lymphocyte gate by the percent of lymphocytes in the lymphocyte gate (based on forward and side light scatter parameters), bearing CD4 or CD8. As shown in Figures 2 and 3, a transient decrease of both CD4+ and CD8+ T-lymphocytes in peripheral blood (lymphopenia) was observed during dosing. The number of lymphocyte recovered to baseline levels within 2 weeks.

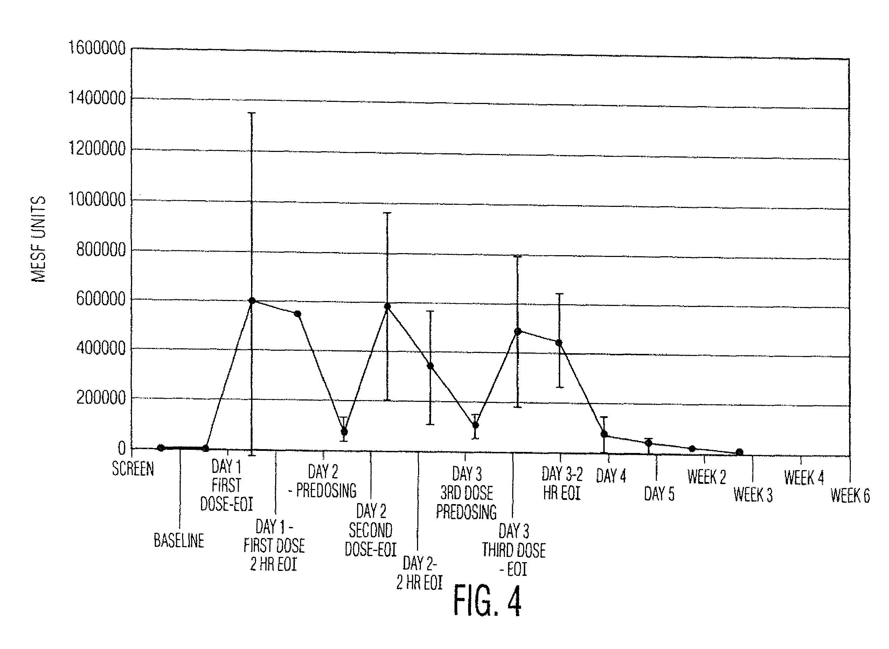

Cell-bound TRX4 was detected on CD4+ and CD8+ T-cells using a fluorescent labeled anti-human IgG antibody reagent. TRX4 was detected on both CD4+ and CD8+ cells 1 hour after administration of each dose of TRX4. Figure 4 depicts detection of cell-bound TRX4 with anti-human IgG as measured in MESF (molecules of equivalent soluble fluorophores) units on CD4+ cells pre- and post-TRX4 dosing. Cell-bound TRX4 decreased after dosing and the values returned to levels close to baseline 24 hours after each dose.

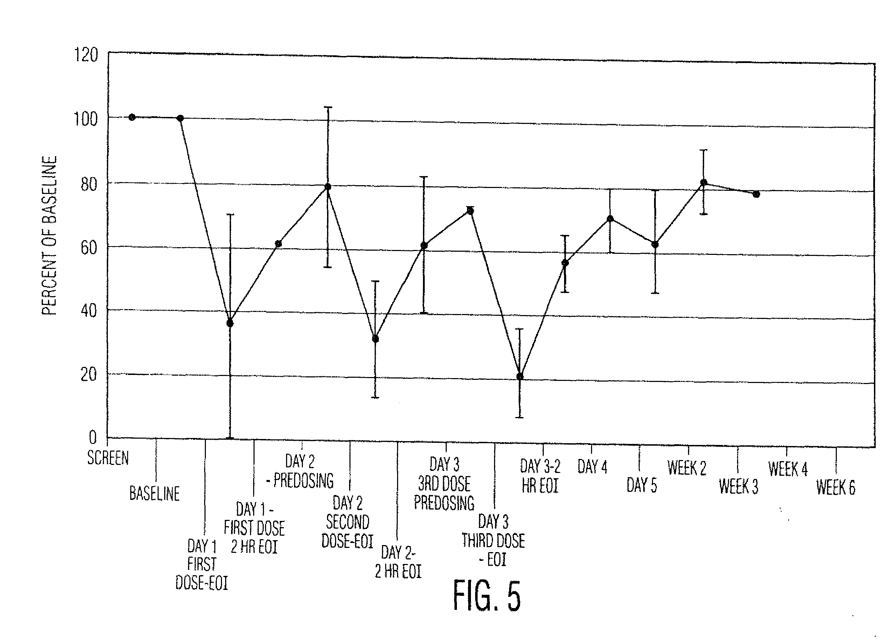

Modulation of the T-cell receptor (TCR) complex was observed on CD4+ and CD8+ T-cells after each dose of TRX4. Figure 5 shows modulation of TCRαβ sites as detected by staining CD4+ T-cells pre- and post-TRX4 dosing with a non-competing TCR antibody labeled with fluorescein isothiocyanate (FITC). The partial modulation returned to approximately 80% of baseline before the second and third doses of TRX4. By three weeks, the levels were approximately 80% of baseline.

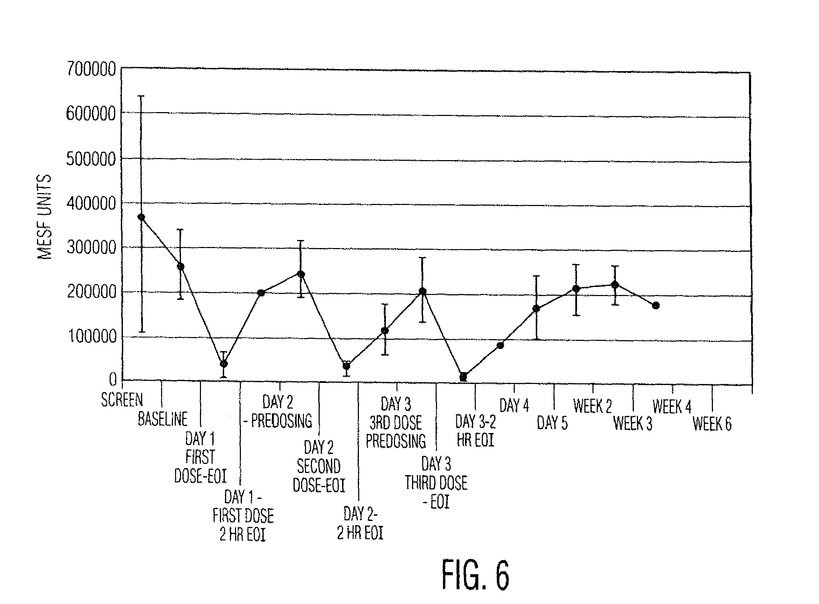

A decrease in the number of free CD3 sites was observed after each dose of TRX4. Figure 6 is a graph of free CD3 sites in CD4+ T-cells as measured in MESF units. The number of free CD3 sites returned to levels close to baseline before the second and third doses of TRX4. The number of free CD3 sites returned to levels close to baseline shortly after the third dose of TRX4 (Day 5).

The above results show that a number of changes in pharmacodynamic parameters were observed. Transient lymphopenia of both CD4+ and CD8+ T-cells occurred after TRX4 administration. In addition, cell-bound antibody was detected, as well as partial modulation of the TCR/CD3 complex and saturation of CD3 after TRX4 administration.

### EXAMPLE 2

A three-day dose escalation study or conditioning regimen with two cohorts of 6 to 8 subjects each is undertaken. Cohort A receives 0.1 mg (about 1.4 µg/kg) of TRX4 antibody on Day 1, 0.3 mg (about 4.3 µg/kg) of TRX4 antibody on Day 2, and 0.5 mg (about 7.1 µg/kg) of TRX4 antibody on Day 3. Cohort B receives 0.3 mg (about 4.3 µg/kg) on Day 1, 0.5 mg (about 7.1 µg/kg) on Day 2, and 1.0 mg (about 14.3 µg/kg) on Day 3.

After the above conditioning regimen is completed, a six- to eight-day dosing regimen is conducted in which each subject is subjected to the same three-day conditioning regimen described in Example 2 for Cohort B, coupled with additional treatment doses. Each subject receives 0.3 mg (4.3 µg/kg) of TRX4 on Day 1, 0.5 mg (7.1 µg/kg) of TRX4 on Day 2 and 1.0 mg (14.3 µg/kg) of TRX4 on each of Days 3 through 8.

While such cohort is being dosed, the doses given on Days 4 through 6 to 8 are escalated to determine the maximum safe dose. Once a maximum safe dose is identified, another dosing cohort in the pivotal trial is added.

The pivotal trial is a randomized, double blind, placebo controlled trial in which more than one dosing regimen arm is compared with the placebo. Follow-up testing for efficacy and safety is conducted at 6, 12, 18, and 24 months, and can be conducted up to 48 months if necessary. Endpoints are mixed meal stimulated C-peptide AUC at 2, 3, and 4 hours post-ingestion, insulin usage, and hemoglobin Alc (HbAlc).

### EXAMPLE 3

### PHARMACOKINETIC AND PHARMACODYNAMIC DATA FROM SUBJECTS DOSED WITH TRX4

In this example, pharmacokinetic (PK) and pharmacodynamic (PD) parameters of TRX4 were evaluated in subjects from four cohorts, which, with the exception of Cohort 4, received various sequences of 3 intravenous doses of TRX4 on consecutive days. In this example, Cohort 1 received 0.1mg TRX4 on each of Days 1, 2, and 3. Cohort 2 received 0.5mg TRX4 on each of Days 1, 2, and 3. Cohort 3 received 0.1mg TRX4 on Day 1, 0.3mg TRX4 on Day 2, and 0.5mg TRX4 on Day 3, Cohort 4 received 0.3mg TRX4. As will be described further hereinbelow, T cell pharmacodynamic parameters included counts of T lymphocytes and lymphocyte subsets in the peripheral blood compartment as well as flow cytometric evaluation of bound TRX4, T cell receptor (CD3/TCR complex) modulation from the cell membrane, and saturation of the CD3/TCR complex by TRX4 on T cells in the peripheral pool. Detectable quantities (≥0.02 µg/mL) of TRX4 most often were observed in samples obtained from subjects given multiple intravenous TRX4 doses of 0.5 mg. Similarly, consistent binding of TRX4 to T cells, modulation and saturation of the CD3/TCR complex, and transient decreases in absolute numbers of peripheral T cells in blood were observed most consistently in cohorts receiving at least one 0.5mg dose. In those cohorts, consistent detectable binding of TRX4 to the T cell membrane and modulation of the CD3/TCR complex and partial saturation persisted for more than 24 hours after the last dose. Taken together, a dosing regimen containing a multiplicity of ≥0.5 mg doses would be predicted to result in TRX4 serum concentrations that are known to inhibit T cell function in vitro while evoking TRX4 binding, partial or complete saturation of the CD3/TCR complex, partial or complete modulation of the CD3/TCR complex, and transient lymphopenia in vivo, all relevant PD parameters thought to be associated with TRX4 function.

### Pharmacodynamic Data Summary

All subjects who received TRX4 in intravenous doses >0.3 mg had rapid, transient decreases in the numbers of lymphocytes (both CD4+ and CD8+ T cells) in the blood, probably caused by redistribution or re-trafficking of T cells in the periphery. For most dosing regimens, CD4+ and CD8+ T cell counts were generally within the normal range (0.53-1.76 × 10⁹/L for CD4+ T cells and 0.30-1.03 × 10⁹/L for CD8+ T cells) with the exception of transient decreases below the normal range in many subjects after doses of 0.3 mg and higher. The decrease in peripheral lymphocytes most likely was a redistribution phenomenon, because in all cohorts lymphopenia was transient, cell numbers had largely returned to at least 90% of baseline by day 14, and no clinical evidence of cell lysis (increased LDH, K+, etc.) was observed. No significant changes were observed in CD19+ B cell counts. The values observed for B cells were within the normal range (0.06-0.66 × 10⁹/L) for all subjects. The PD changes observed during and immediately after TRX4 infusions were consistent with what was observed in the Phase II study conducted in the EU (8 mg × 6) (Keymeulen, et al. N, Engl. J. Mend, Vol. 352, pgs 2598-2608 (June 23, 2005)), although the degree of lymphopenia was less pronounced in this example and was of a shorter duration, consistent with the lower doses of TRX4 being administered than in the EU study. (Keymeulen, 2005).

Cell-bound TRX4 was detected in all cohorts in a dose-dependent manner. Cell-bound TRX4 was detected for 48 hours after doses of 0.5 mg. Partial CD3/TCR modulation was evident in all cohorts, with the exception of subjects who received 3 × 0.1 mg, after TRX4 administration. CD3/TCR modulation largely was dose dependent. Modulation of 80% or greater was observed after doses of 0.5 mg. Partial modulation also persisted for 24 hours after these doses. Modulation was measured indirectly in the EU study; the data presented here from this Example show a lower degree of CD3/TCR modulation and a shorter duration of CD3/TCR modulation than in the EU study, consistent with the lower doses of TRX4.

A decrease in free binding sites (indicating CD3 saturation by TRX4) was evident and coincident with the detection of TRX4 bound to T cells. The extent and duration of CD3 saturation were largely dose dependent. Incomplete saturation was present until Day 5 in the cohorts that received 3 doses of TRX4 (with the exception of 0.1 mg × 3).

### PHARMACOKINETIC DATA

### Determination of TRX4 Serum Levels

Serum levels of TRX4 were determined by an ELISA assay conducted under good laboratory practices, or GLP. Blood samples were collected before infusion of TRX4, at the end of each infusion, and 2 hours after the end of infusion. The ELISA assay uses two anti-TRX4 monoclonal antibodies, one as the capture antibody and the second as the bridging antibody. The limit of quantitation (LOQ) of this assay is 0.0199 µg/mL. The results are shown in Table 1 below.

Serum concentrations of TRX4 for the majority of samples from all cohorts were either below or just above the LOQ. Because of the limited number of samples with TRX4 concentrations above the LOQ, the limited number of subjects assessed, and intersubject variability, a pharmacokinetic analysis and definitive conclusions concerning product disposition could not be made. Nevertheless, the greatest number of samples containing detectable levels of TRX4 were from Cohort 2, the only cohort in which subjects were given a multiplicity of the 0.5 mg dose (Cohort 2). In this cohort, maximum serum concentrations at the end of infusion approached 0.10 µg/mL in some subjects, but this finding was not consistent in each subject nor observed after each dose. Nadir serum concentrations prior to the third 0.5 mg dose were 0.02 - 0.03 µg/mL in two of three subjects, while the third subject had undetectable levels. Given the limited data from the other cohorts, meaningful comparative observations could not be drawn.

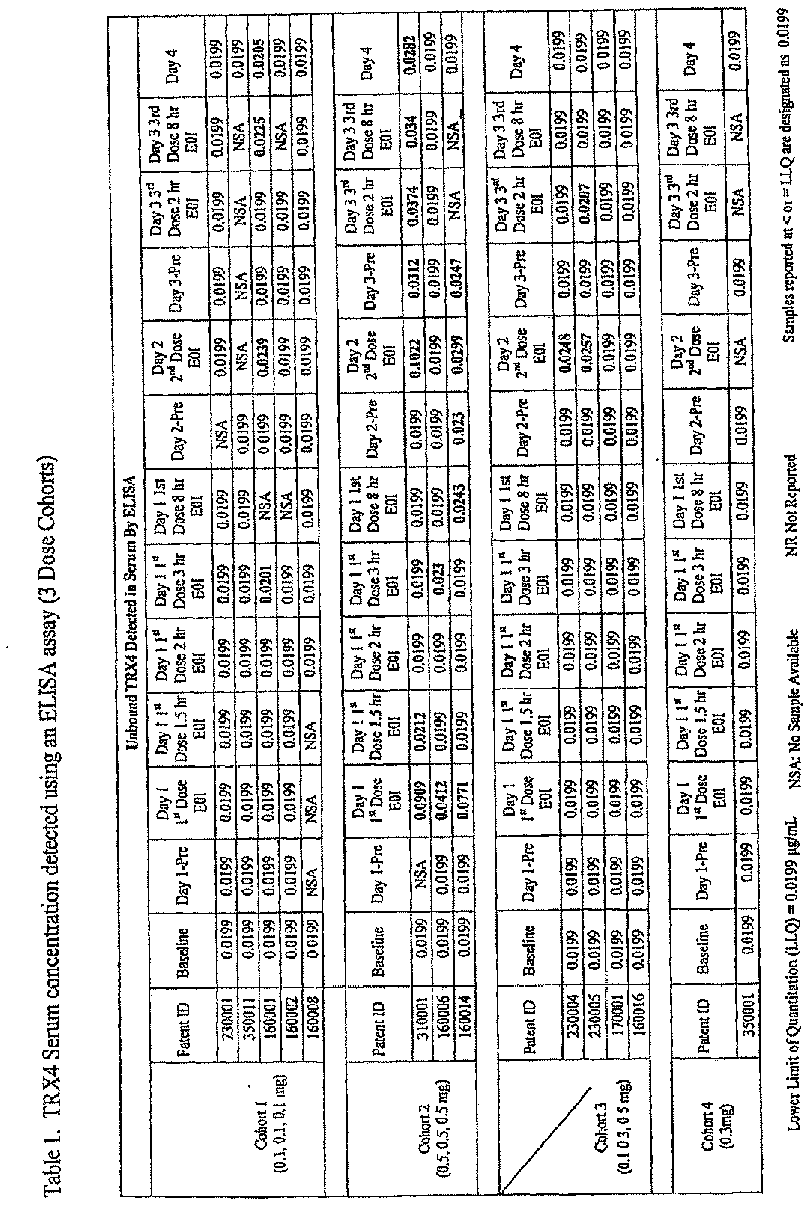

### Pharmacodynamic assessment in subjects dosed with TRX4

### Flow Cytometry Analysis of Lymphocyte Subsets, Cell-bound TRX4, and CD3/T cell Receptor Complex Saturation and Modulation

### Assessment of Circulating Lymphocyte Phenotype and Number

Flow cytometry immunophenotyping was used to monitor changes in peripheral blood lymphocytes and subsets of total T cells, CD4+ T cells and CD8+ T cells. CD19+ B cells also were monitored as B cells are a target of Epstein Barr virus (EBV), and EBV reactivation was seen in the Phase II study conducted in the EU. (Keymeulen, 2005). Natural Killer (NK) cells were monitored in the 3 dose cohorts; no significant changes in NK levels were observed. Activated T cells, defined as T cells expressing CD25, CD38, CD69, or HLA-DR were monitored in the 3 dose cohorts; no increase in activated T cells was observed in any subject after TRX4 administration.

### Method of Calculation of Absolute Lymphocyte Counts

Absolute counts for each lymphocyte subset per liter were calculated based on CD markers by multiplying the absolute number of lymphocytes per liter by the percentage of lymphocytes in the lymphocyte flow cytometry gate (as determined using forward and side light scatter parameters) bearing the CD marker of interest. To facilitate accurate enumeration of lymphocyte populations that occur at low frequencies, 50,000 events were collected by flow cytometry. The absolute number of lymphocytes was determined by multiplying the total white blood cell (WBC) count (from a hematology sample taken at the same time as the flow cytometry sample) by the percentage of lymphocyte as determined by the WBC differential cell count. Absolute counts and percentages were calculated for each parameter, and changes from baseline were determined for each post-baseline assessment.

### Detection of TRX4 Bound to CD4+ and CD8+ T cells

Cell-bound TRX4 was detected on CD4+ and CD8+ T cells using an anti-human IgG antibody reagent. Fluorescence intensity was quantified by using standard units known as Molecules of Equivalent Soluble Fluorochrome (MESF). MESF units were determined by comparing the fluorescence intensity signal from a microbead standard to the signal from the sample solution stained with the same fluorochrome. There is a direct relationship between the MESF value of a cell population and the number of binding antibodies. Use of MESF standardizes data collected on different days. The MESF of the anti-human IgG was used to quantify the amount of cell-bound TRX4.

### CD3/T Cell Receptor (TCR) Complex Analysis: CD3/TCR Modulation and Saturation

CD3 is one of the components of the TCR complex. CD4+ T cells express Approximately twice the number of CD3/TCR complexes as CD8+ T cells. To evaluate the level of surface expression of the CD3/TCR complex and its modulation, αß TCR expression was determined for both CD4+ and CD8+ T cells using the antibody BMA031. (Abcam. Inc., Cambridge, MA; Borst, et al., Hum. Immunol., Vol. 29, pgs. 175-188 (1990)). Binding of this antibody is not blocked by TRX4 bound to the CD3 surface molecule when TRX4 serum levels are below 1 µg/mL. Serum levels greater than I µg/mL were not detected in any of subjects described in this example. The MESF of the anti-TCR αß antibody was used to quantify the number of CD3/TCR complexes present on T cells.

Free TRX4 binding sites (unoccupied by previously administered TRX4) were detected by staining with biotinylated TRX4. The MESF of bound biotinylated TRX4 is directly proportional to the availability of free TRX4 binding sites.

### Cohort Summaries:

Unless noted, the changes in PD parameters observed were similar in CD4+ and CD8+ T cells.

### Cohort One (0.1 mg x 3) (n=4):

### Lymphocyte Numbers

Circulating T cells and B cells did not change significantly after TRX4 dosing in this cohort.

### Detection of TRX4 Bound to CD4+ T cells

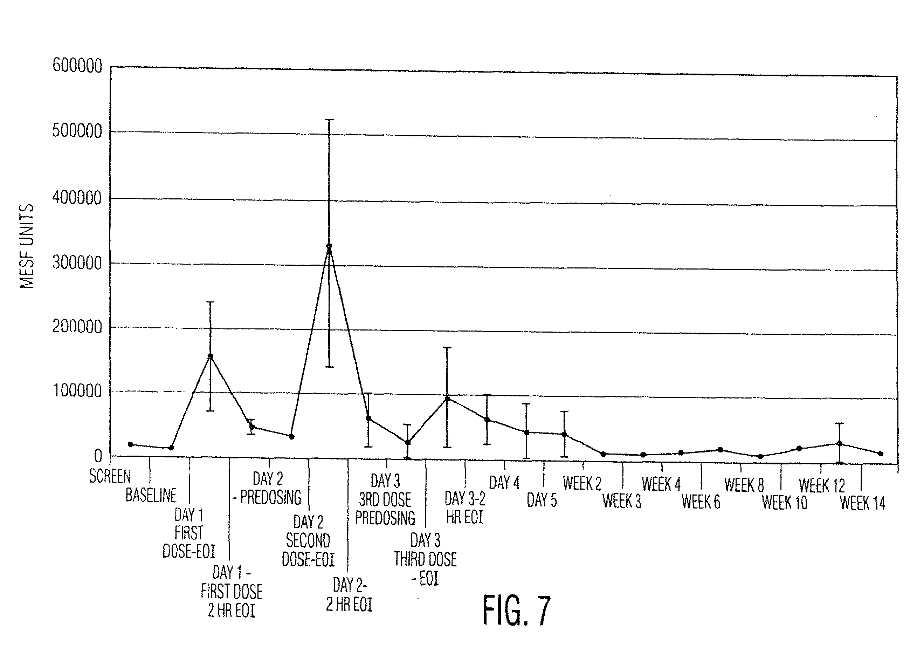

Maximum amounts of TRX4 bound to CD4+ T cells were detected at the end of the TRX4 infusion (Figure 7). Peak amounts occurred after the second dose of TRX4 (300,000 MESF units), although bound TRX4 was appreciably lower than what was observed in cohorts with higher doses. Bound TRX4 decreased on all T cells after Day 3, and no bound TRX4 was detected on the surface ofT cells by Day 14.

### T cell Receptor Analysis

No significant modulation of the CD3/TCR receptor complex (that is, no decrease in MESF of TCR αß) was observed on CD4+ T cells after dosing with TRX4.

A slight and transient decrease in free CD3 sites was evident in CD4+ T cells at the end of the first infusion of TRX4; free CD3 sites returned to baseline levels 2 hours after the end of infusion. Approximately 37% saturation of CD3 was observed at the end of infusion with recovery to baseline levels by 2 hours after the end of infusion. No significant decrease in free CD3 sites occurred after the second or third dose of TRX4. Free CD3 sites remained at baseline levels for the remainder of the study.

### Cohort Two (0.5 mg x 3) (n=3; 2 subjects received 3 doses, 1 subject received 2 doses):

### Lymphocyte Numbers

Circulating T cell counts were reduced in all subjects after the first infusion of TRX4 and remained below baseline levels during the 3 days of dosing. Lymphocyte counts began to increase at Day 4 and were close to baseline levels by Day 5. No significant changes were observed in circulating B cells.

### Detection of TRX4 Bound to CD4+ T cells

Maximum TRX4 binding was detected on CD4+ T cells at the end of the TRX4 infusion on each day of dosing. The amount of binding (approximately 600,000 MESF units after each dose) was greater than what was observed in Cohort 1, consistent with a higher dose of TRX4. Cell-bound TRX4 was still present (400,000 MESF units) 2 hours after the end of dosing. TRX4 binding returned to levels close to baseline and was generally not detected prior to the second and third doses; however, TRX4 binding was detected on the surface of CD4+ T cells after the third dose until Day 5.

### T Cell Receptor Analysis

Significant CD3/TCR modulation was observed on CD4+ T cells at the end of each TRX4 infusion. Approximately 60% modulation was observed after the first dose, approximately 70% modulation after the second dose and 80% modulation after the third dose. Approximately 40% modulation was observed 2 hours after the end of infusion at all 3 doses. No modulation was evident before the second dose. Approximately 20% modulation was evident prior to the third dose. After the third dose, modulation was evident until Day 14.

A decrease in free CD3 sites was evident on CD4+ T cells at the end of each infusion of TRX4. Approximately 90% saturation was observed after the first dose, approximately 80% saturation after the second dose and 95% saturation after the third dose. Approximately 55% saturation was observed 2 hours after the end of infusion at all 3 doses. Significant saturation was not evident prior to the second and third doses. After the third dose, partial saturation was observed until Day 5.

### Cohort Three (0.1 mg, 0.3 mg, 0.5 mg) (n=4):

### Lymphocyte Numbers

Circulating T cells were modestly reduced in all subjects after the 0.3 mg and 0.5 mg doses of TRX4. Lymphocyte counts began to increase at Day 4 and were close to baseline levels by Day 5. No significant changes were observed in circulating B cells.

### Detection of TRX4 Bound to CD4+ T cells.

Cell-bound TRX4 was detected on CD4+ T cells at the end of the TRX4 infusion on each day of dosing. TRX4 binding returned to levels dose to baseline between doses and generally was not detected prior to the second or third dose. The increase in cell-bound TRX4 occurred in a dose dependent manner with the greatest amount of TRX4 detected after the 0.5 mg dose (100,000 MESF units after 0.1 mg, 300,000 MESF units after 0.3 mg and 800,000 MESF units after 0.5 mg). TRX4 was no longer detected on the surface ofT cells by Day 4.

### T Cell Receptor Analysis

CD3/TCR modulation was observed on CD4+ T cells. Modulation was dose dependent with 15% modulation observed after 0.1 mg, 50% modulation after 0.3 mg and 80% modulation after 0.5 mg. CD3/TCR expression recovered to levels close to baseline between dosing. TCR modulation was not detectable after Day 5.

There were dose dependent decreases in free CD3 sites on CD4+ T cells at the end of each infusion of TRX4. Transient partial saturation was observed after 0.1 mg (45%) and 0.3 mg (70%). Free CD3 sites returned to close to baseline levels 2 hours after the end of the first 2 infusions. After 0.5 mg, 90% saturation was observed at the end of infusion, and 45% saturation was still evident 2 hours after the last infusion. No significant decrease in free CD3 sites was observed after Day 5.

### Lymphocyte Counts

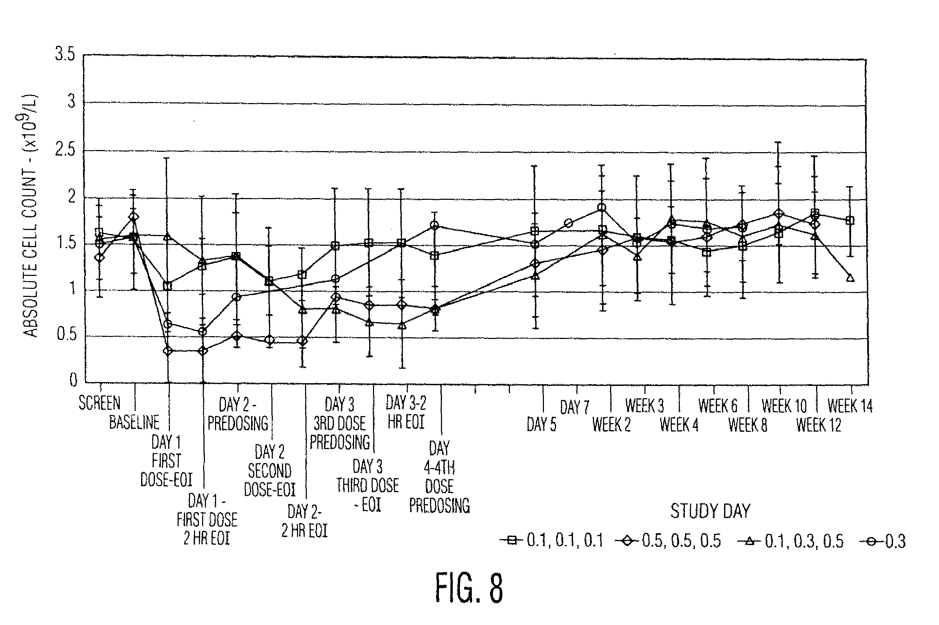

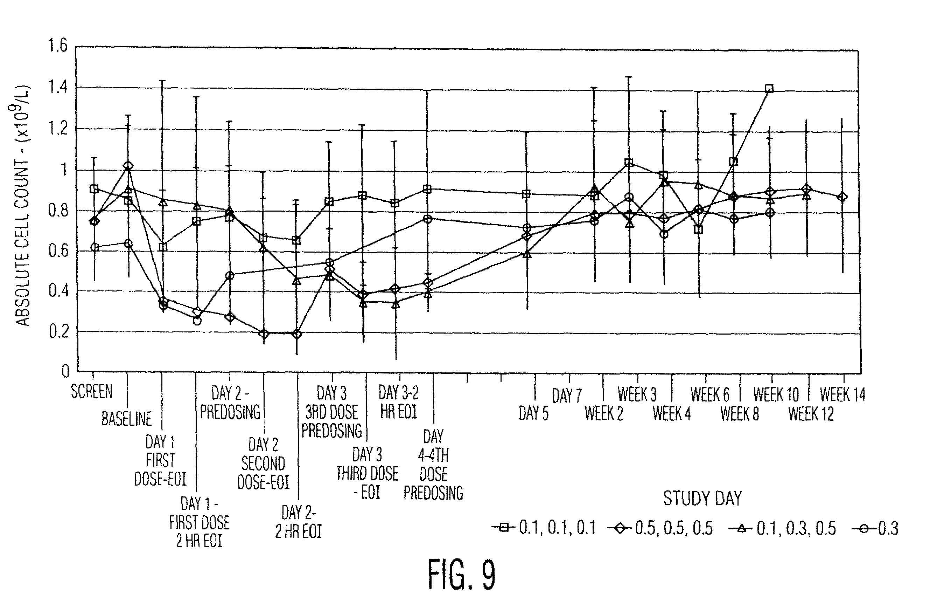

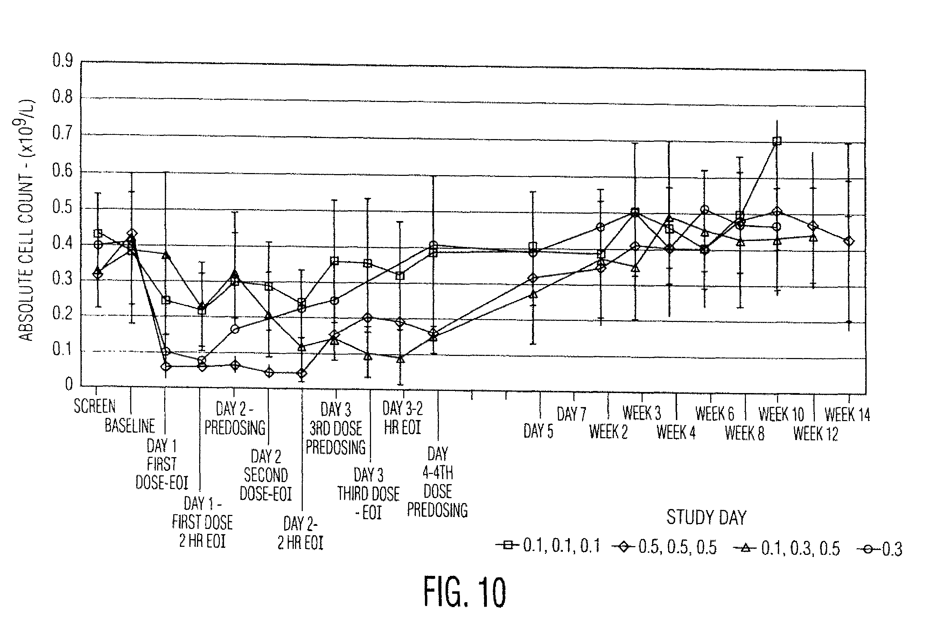

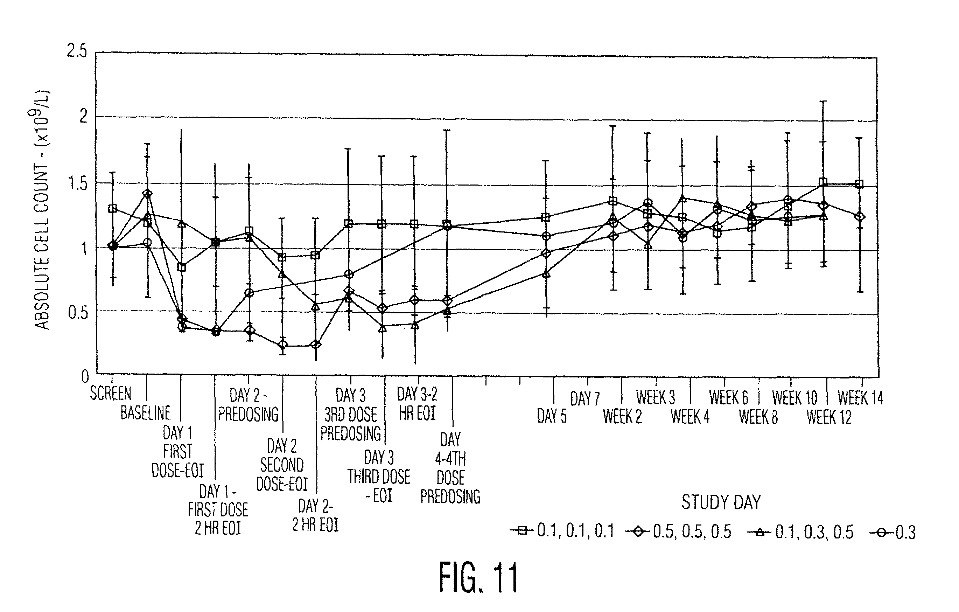

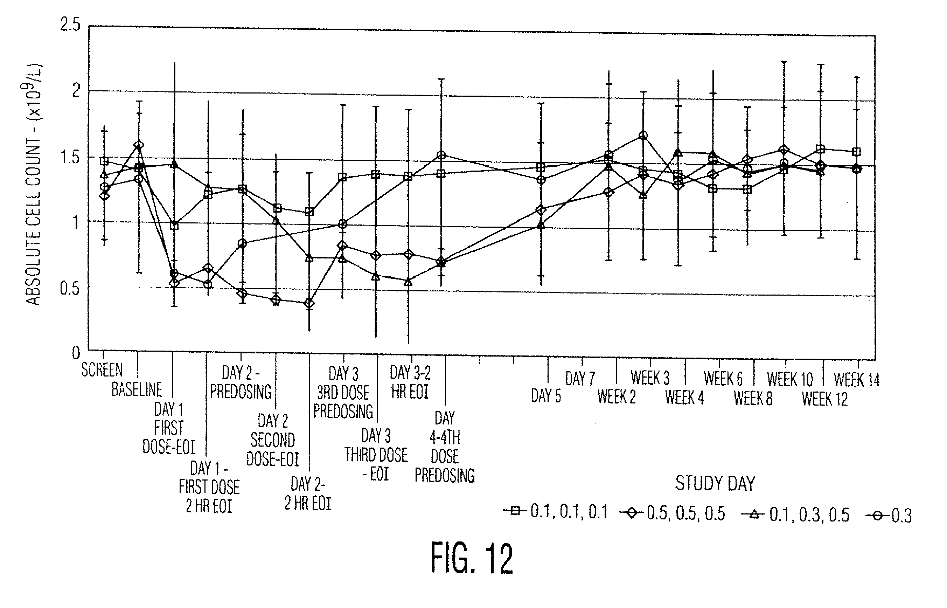

The mean absolute lymphocyte count, CD4+ lymphocyte count, CD8+ lymphocyte count, TCR+ (CD4+CD8+) lymphocyte count, and CD2+ lymphocyte count were measured for up to 14 weeks after the start of the treatment for each of the cohorts. Figure 8 shows the median total number of all lymphocytes for each of the four cohorts up to 14 weeks after treatment. Figure 9 shows the median total number of CD4+ T-lymphocytes for each of the four cohorts up to 14 weeks after treatment. Figure 10 shows the median total number of CD8+ T-lymphocytes for each of the four cohorts up to 14 weeks after treatment. Figure 11 shows the median total number of TCR+(CD4+ and CD8+) T-lymphocytes for each of the four cohorts up to 14 weeks after treatment. Figure 12 shows the median total number of CD2+ T-lymphocytes for each of the four cohorts up to 14 weeks after treatment.

The lymphocyte counts for all cohorts had returned to at or near baseline levels within approximately 5 days after the start of the treatment, and remained at or near such baseline levels.

### EXAMPLE 4

In this example, pharmacokinetic and pharmacodynamic parameters of TRX4 were evaluated in subjects from four cohorts that received various sequences of 4 intravenous doses of TRX4 on consecutive days. Cohort A*, which included one patient, received 0,05mg TRX4 on Day 1, 0.1mg TRX4 on Day 2, 0.15mg on Day 3, and 0.25mg on Day 4. Cohort A, which included 4 patients, received 0.1mg TRX4 on Day 1, 0.2mg TRX4 on Day 2, 0.3mg on Day 3, and 0.5mg on Day 4. Cohort B, which included 4 patients, received 0.1mg TRX4 on Day 1, 0.2mg TRX4 on Day 2, 0.3mg on Day 3, and 0.75mg on Day 4. Cohort C, which included one patient, received 0.1mg TRX4 on Day 1, 0.2mg TRX4 on Day 2, 0.3mg on Day 3, and 1.0mg on Day 4.

As in Example 3, T-cell pharmacodynamic parameters included counts of T-lymphocytes and lymphocyte subsets in the peripheral blood compartment as well as flow cytometric evaluation of bound TRX4, T cell receptor (CD3/TCR complex) modulation from the cell membrane, and saturation of the CD3/TCR complex by TRX4 on T cells in the peripheral pool. As in Example 3, consistent binding of TRX4 to T cells, modulation and saturation of the CD3/TCR, and transient decreases in absolute numbers of peripheral T cells in blood were observed most consistently in cohorts receiving at least one 0.5mg dose. In those cohorts, consistent binding of TRX4 to the T cell membrane and modulation of the CD3/TCR and partial saturation persisted for more than 24 hours after the last dose.

Also, all subjects who received TRX4 in intravenous doses >0.3mg had rapid, transient decreases in the numbers of lymphocytes (both CD4+ and CD8+ cells) in the blood. As in Example 3, for most dosing regimens, CD4+ and CD8+ T cell counts generally were within the normal range (0,53-1.76X 10⁹/l for CD4+ T cells and 0.30-1.03X 10⁹/l for CD8+ T cells) with the exception of transient decreases below the normal range in many subjects after doses of 0.3 mg and higher. The decrease in peripheral blood lymphocytes, as in Example 3, most likely was a redistribution phenomenon, because in all cohorts lymphopenia was transient, cell numbers largely had returned to at least 90% of baseline by Day 14, and no clinical evidence of cell lysis (increased LDH,K+ etc.) was observed. Also, no significant changes were observed in CD19+ B cell counts. The values observed for B cells were within the normal range (0.06-0.66 X 10⁹/l) for all subjects. The pharmacodynamic changes observed during and immediately after TRX4 infusions were consistent with what was observed in the Phase II study conducted in Europe (8mg/day for 6 days) (Keymeulen, 2005), although the degree of lymphopenia was less pronounced in this example and was of a shorter duration, which is consistent with the lower doses of TRX4 being administered than in the European study (Keymeulen, 2005).

As in Example 3, cell-bound TRX4 was detected in all cohorts in a dose-dependent manner. Cell-bound TRX4 was detected for 48 hours after doses of 0.5 mg and higher. Partial CD3/TCR modulation was evident in all cohort after TRX4 administration. CD3/TCR modulation was largely dose dependent. Modulation of 80% or greater was observed after doses of 0.5 mg and higher. Partial modulation also persisted for 24 hours after these doses. Modulation was measured indirectly in the EU study (Keymeulen, 2005); the data presented here in this example show a lower degree of CD3/TCR modulation and a shorter duration of CD3/TCR modulation than in the EU study, consistent with the lower doses of TRX4,

A decrease in free TRX4 binding sites (indicating CD3 saturation by TRX4) was evident and coincident with the detection of TRX4 bound to T cells. The extent and duration of CD3 saturation were largely dose dependent. Incomplete saturation was present until Day 5 in the lowest 4-dose cohort (0.05 mg, 0.1 mg, 0.15 mg and 0.25 mg). In the other 4-dose cohorts, saturation of 90% or greater was observed after doses of 0.5 mg and higher and partial saturation (Approximately 40%) was present at 24 hours after the infusion. Incomplete saturation was present until Week 3 in subjects who received 4 doses of TRX4 (0.1 mg, 0.2 mg, 0.3 mg and 0.5 mg). Saturation was present until Day 7 in subjects who received 4 *doses* of TRX4 with a fourth dose of either 0.75 mg or 1 mg.

### Pharmacokinetic Data

### Determination of TRX4 Serum Levels

Serum levels of TRX4 were determined using the ELISA assay described in Example 3. As in Example 3, the limit of quantitation (LOQ) of the assay was 0.0199µg/ml. The results are shown in Table 2 below.

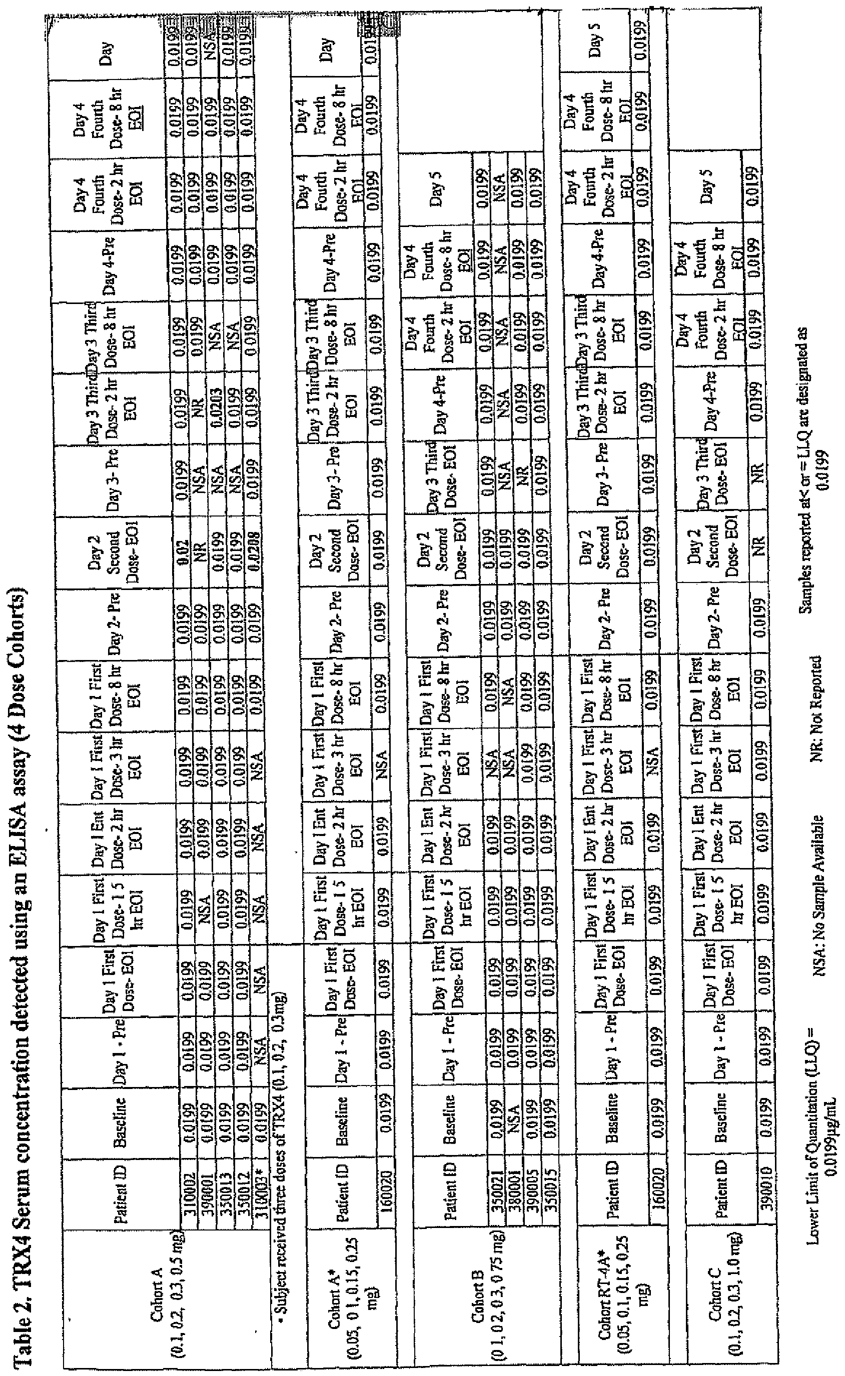

### Pharmacodynamic Assessment in Subjects Dosed with TRX4: Flow Cytometry Analysis of Lymphocyte Subsets. Phenotype, and Numbers; Cell Bound TRX4; and CD3/T Cell Receptor Complex Saturation and Modulation

Flow cytometry immunophenotyping was used as in Example 3 to monitor changes in peripheral blood lymphocytes and subsets of total T-cells, CD4+ T-cells and CD8+ T-cells, and CD 19+ B cells also were monitored.

Absolute counts for lymphocytes and for lymphocytes subsets were calculated based on CD markers as described in Example 3. Absolute counts and percentages were calculated for each parameter, and changes from baseline were determined for each post-baseline assessment.

Cell-bound TRX4 was detected on CD4+ and CD8+ T-cells using an anti-human IgG antibody reagent, and fluorescence intensity was quantified using standard MESF units as described in Example 3.

In order to evaluate the level of surface expression of the CD3/TCR complex and its modulation, αß TCR expression was determined for both CD4+ and CD8+ T-cells using the antibody BMA031, as described in Example 3. The MESF of the anti-TCR αß antibody was used to quantify the number of CD3/TCR complexes present on T-cells.

Free TRX4 binding sites (unoccupied by previously administered TRX4) were detected by staining with biotinylated TRX4 . The MESF of bound biotinylated TRX4 is directly proportional to the availability of free TRX4 binding sites.

### Cohort A* (0.05 mg, 0.1 mg, 0.15 mg, 0.25 mg) (n=1):

### Lymphocyte Numbers

Circulating T cell counts were reduced modestly after the first dose of TRX4. Lymphocyte counts returned to baseline levels at Day 14. A transient decrease in circulating B cells was observed on the second and third days of dosing.

### Detection of TRX4 Bound to CD4+ T cells

Cell-bound TRX4 was detected on CD4+ T cells at the end of the TRX4 infusion on each day of dosing (300,000 MESF units after 0.05 mg, 700,000 MESF units after 0.1 mg, 800,000 MESF units after 0.15 mg and 80,000 MESF units after 0.25 mg). The greatest amounts of cell-bound TRX4 were detected after the second and third doses. Only a slight increase in cell-bound TRX4 was detected after the fourth dose which was likely due to the simultaneous occurrence of TCR modulation. TRX4 was no longer detected on the surface of T cells by Day 5 on either cell subset.

### T cell Receptor Analysis

CD3/TCR modulation was observed on CD4+ T cells in a somewhat dose dependent manner with 45% modulation after 0.05 mg, 20% modulation after 0.1 mg, 25% modulation after 0.15 mg and 80% modulation after 0.25 mg. CD3/TCR modulation was no longer detectable by Day 5 and subsequent levels were close to baseline.

A decrease in free CD3 sites was evident on CD4+ T cells after each dose of TRX4 and this occurred in a dose dependent manner with 50% saturation after 0.05 mg, 70% saturation after 0.1 mg, 80% saturation after 0.15 mg, and 95% saturation after 0.25 mg. This returned to levels close to baseline levels 2 hours after the end of each infusion. No significant decrease in free CD3 sites was observed after Day 5.

### Cohort A (0.1 mg, 0.2 mg, 0.3 mg, 0.5 mg) (n=4):

### Lymphocyte Numbers

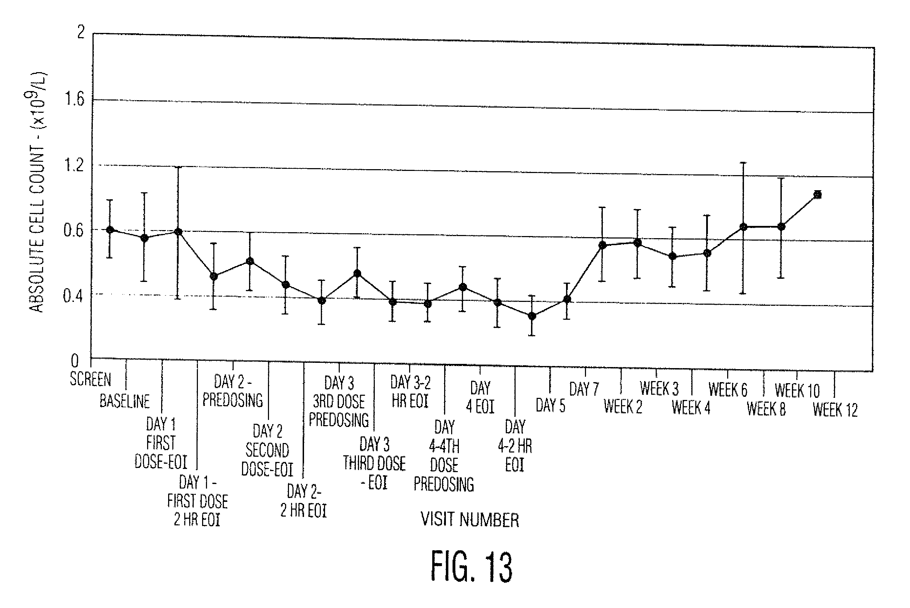

Circulating CD4+ T cells counts were reduced modestly after the first dose of TRX4 and remained so until Day 7 when they returned to baseline (Figure 13). No significant changes were observed in circulating B cells.

### Detection of TRX4 Bound to CD4+ T cells

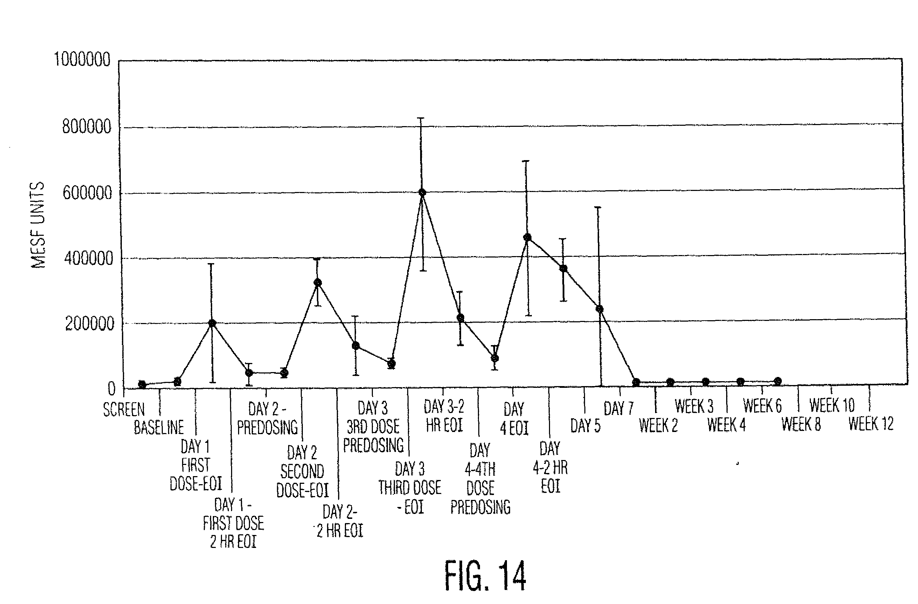

Cell-bound TRX4 was detected on CD4+ T cells at the end of the TRX4 infusion on each day of dosing (200,000 MESF units after 0.1 mg, 375,000 MESF units after 0.2 mg, 600, 000 MESF units after 0.3 mg and 450,000 MESF units after 0.5 mg) (Figure 14). The greatest amount of cell-bound TRX4 was detected after the third and fourth doses of TRX4. Cell-bound TRX4 was still present 2 hours after the end of the 0.5 mg infusion (400,000 MESF units) with detectable TRX4 still present approximately 24 hours later. TRX4 was no longer detected on the surface of T cells by Day 7.

### T cell Receptor Analysis

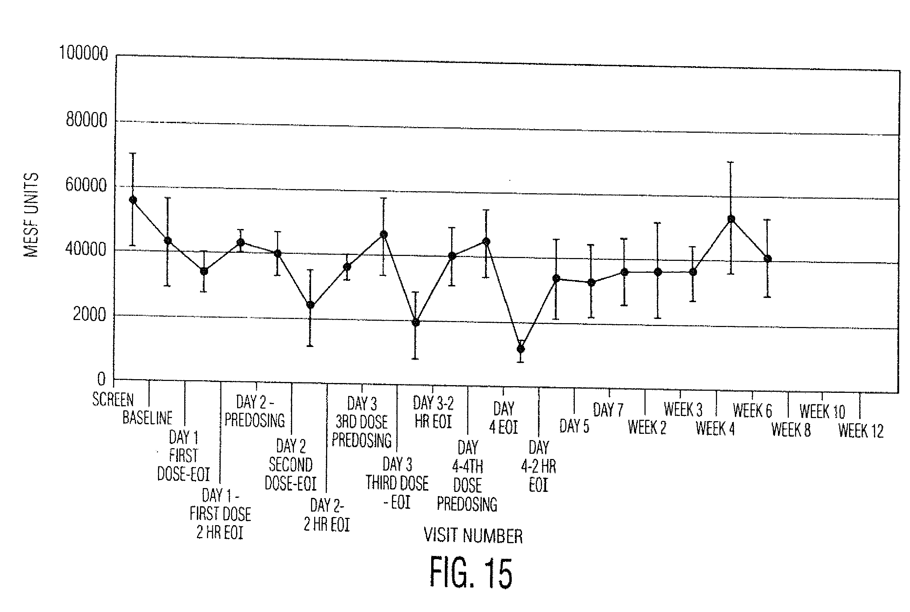

CD3/TCR complex modulation was observed on CD4+ T cells after dosing (Figure 15). Modulation was dose dependent with 45% modulation after 0.1 mg, 40% modulation after 0,2 mg. 60% modulation after 0.3 mg and 80% modulation after 0.5 mg. The degree of modulation was most consistent between subjects at the 0.5 mg dose. CD3/TCR expression recovered to levels close to baseline between doses so that no modulation was evident prior to the second, third, and fourth doses; however, after the fourth dose of 0.5 mg, partial CD3/TCR modulation was evident until Week 3, after which CD3/TCR levels returned to baseline.

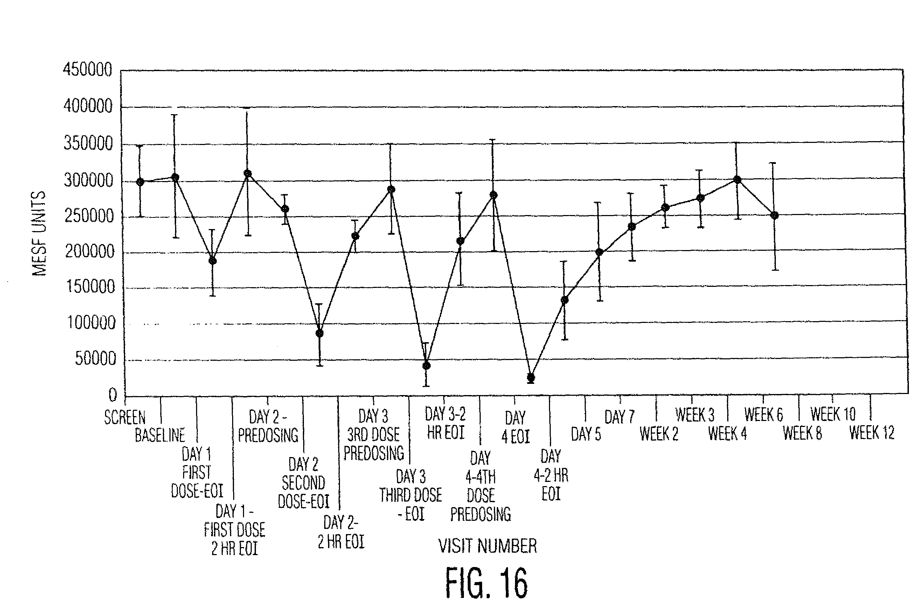

There were dose-dependent decreases in free CD3 sites on CD4+ T cells after each infusion with 40% saturation after 0.1 mg, 70% saturation after 0.2 mg, 85% saturation after 0.3 mg and 95% saturation after 0.5 mg (Figure 16). 60% saturation was evident 2 hours after the end of the last infusion and 35% saturation still was evident the next day.

### Cohort B (0.1 mg, 0.2 mg, 0.3 mg, 0.75 mg) (n=4):

### Lymphocyte Numbers

Circulating T cell counts were reduced modestly after the first dose and remained so until Day 7 when they returned to baseline. No significant changes were observed in circulating B cells.

### Detection of TRX4 Bound to CD4+ T cells

Cell-bound TRX4 was detected on CD4+ T cells at the end of the TRX4 infusion on each day of dosing (2000,000 MESF units after 0.1 mg, 400,000 MESF after 0.2mg,

420,000 MESF units after 0.3 mg and 480,000 MESF units after 0.75 mg). The greatest amount of cell-bound TRX4 was detected after the third and fourth doses. Cell-bound TRX4 was still present 2 hours after the end of the 0.75 mg infusion (550,000 MESF units) with detectable TRX4 still present approximately 24 hours later (200,000 MESF units). TRX4 was no longer detected on the surface of T cells by Day 7.

### T Cell Receptor Analysis

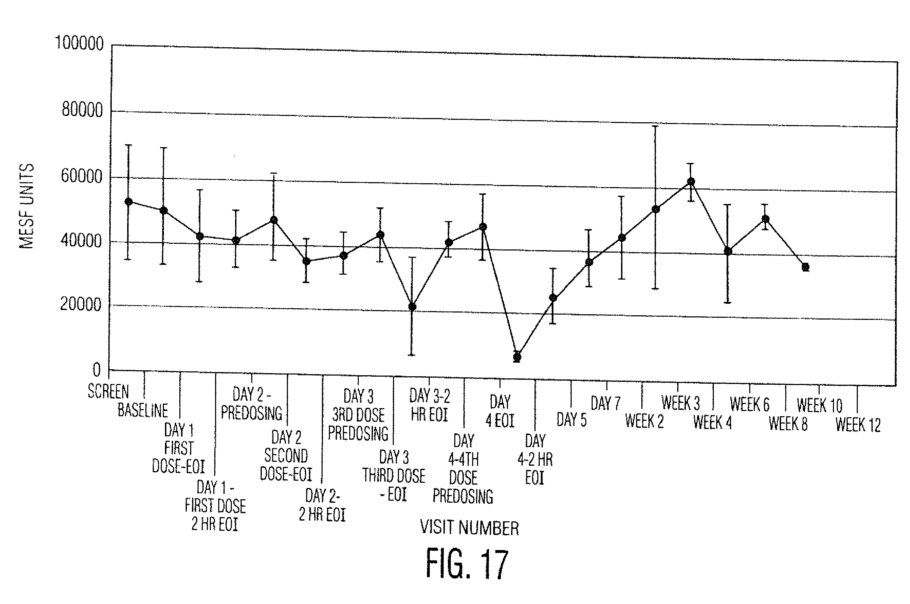

CD3/TCR modulation was observed on CD4+ T cells after dosing (Figure 17). Modulation was dose dependent with 20% modulation after 0.1 mg, 30% modulation after 0.2 mg, 60% modulation after 0.3 mg and 85% modulation after 0.75 mg. Modulation recovered to levels close to baseline between doses so that no modulation was evident prior to the second, third and fourth doses, however, after the fourth dose of 0.75 mg, 50% modulation was still evident 2 hours after the end of the last infusion. Partial CD3/TCR modulation was evident until Week 2, after which CD3/TCR levels remained close to baseline.

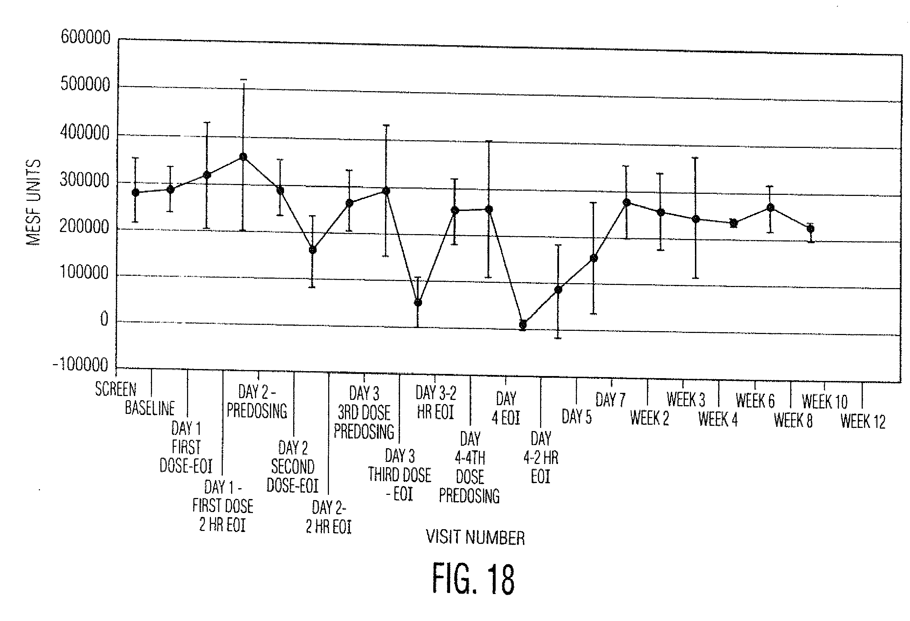

There were dose-dependent decreases in free CD3 sites on CD4+ T cells at the end of each infusion. No significant saturation was detected after 0.1 mg, with 60% saturation after 0.2 mg, 80% saturation after 0.3 mg and 95% saturation after 0.75 mg (Figure 18). 70% saturation was evident 2 hours after the end of the last infusion, and 40% saturation was still evident the next day (Day 5). A full return to baseline levels occurred by Day 7.

### Cohort C (0.1 mg, 0.2 mg, 0.3 mg, 1.0 mg) (n=1):

### Lymphocyte Numbers

Circulating CD4+ T cells counts were reduced modestly at the end of each infusion of TRX4 but returned to levels close to baseline within 24 hours. No significant changes were observed in circulating B cells.

### Detection of TRX4 Bound to CD4+ T cells

Cell-bound TRX4 was detected on CD4+ T cells at the end of the TRX4 infusion on each day of dosing (800,000 MESF units after 0.1 mg, 1,200,000 MESF units after 0.2 mg, 600, 000 MESF units after 0.3 mg and 750,000 MESF units after 1.0 mg). The greatest amount of cell-bound TRX4 was detected after the second dose. Cell-bound TRX4 was still present 2 hours after the end of the 1.0 mg infusion (600,000 MESF units) with detectable TRX4 still present approximately 24 hours later (200, 000 MESF units). TRX4 was no longer detected on the surface of T cells by Day 7.

### T cell Receptor Analysis

CD3/TCR modulation was observed on CD4+ T cells after dosing. Modulation was dose dependent with 25% modulation after 0.1 mg, 75% modulation after 0.2 mg, 85% modulation after 0.3 mg and 88% modulation after 1.0 mg. Modulation recovered to levels close to baseline between dosing so that no modulation was evident prior to the second, third and fourth doses, 50% modulation was still evident 2 hours after the end of the last infusion, and this was still evident on Day 5. CD3/TCR levels appeared to be returning to baseline by Day 7.

Decrease in free CD3 sites were evident on CD4+ T cells at the end of each infusion and occurred in a dose dependent manner with 50% saturation after 0.1 mg, 95% saturation after 0.2 mg, 99% saturation after 0.3 mg and 97% saturation after 1.0 mg. 50% saturation was evident 2 hours after the end of the last infusion, and 30% saturation was still evident the next day (Day 5). A full return to baseline levels occurred by Day 7.

### Lymphocyte Counts

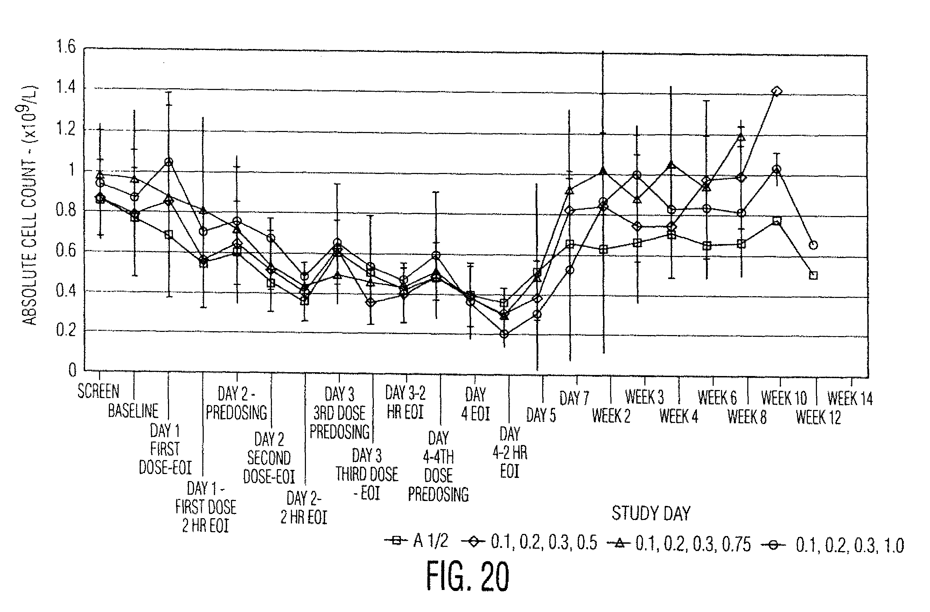

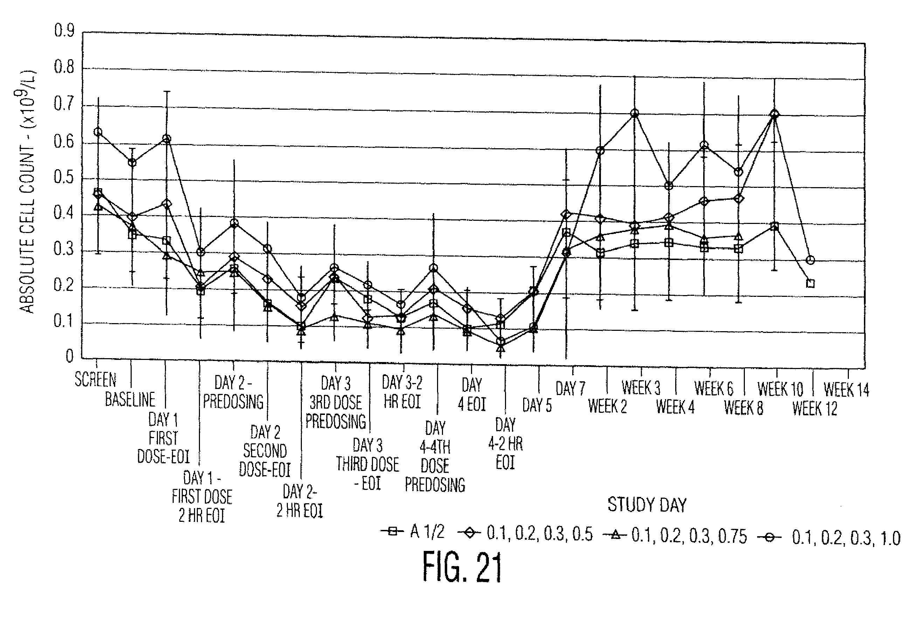

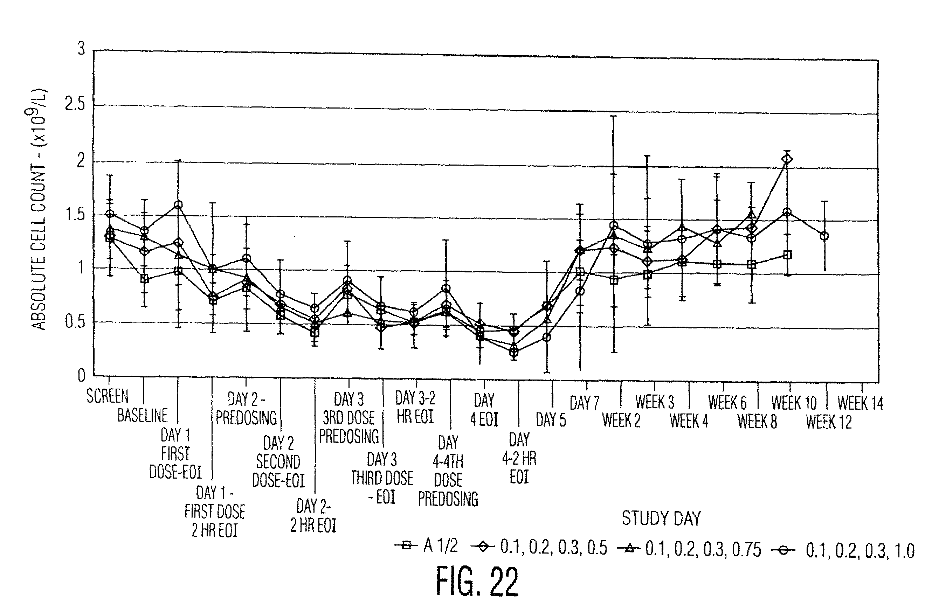

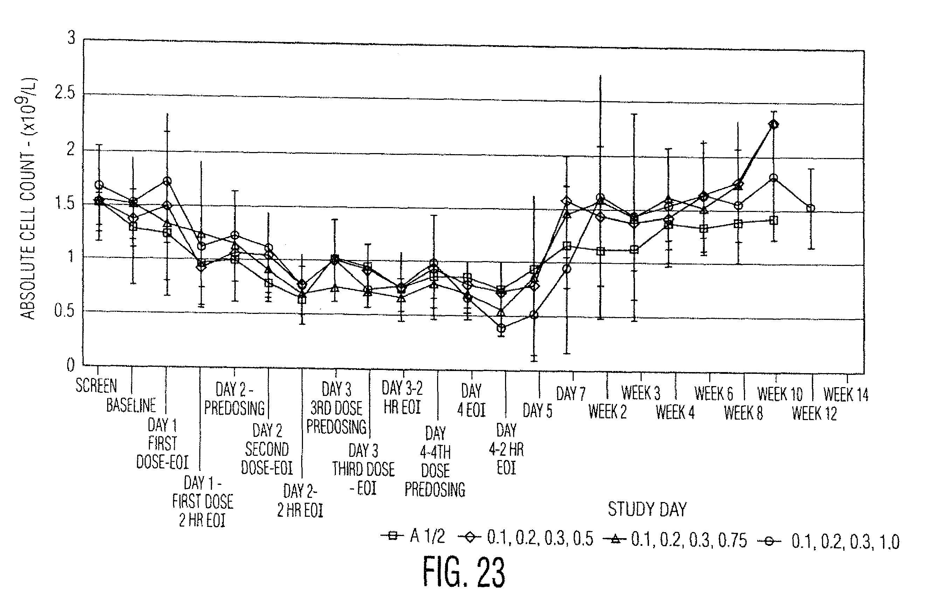

The mean absolute lymphocyte count, CD4+ lymphocyte count, CD8+ lymphocyte count, TCR + (CD4+CD8+) lymphocyte count, and CD2+ lymphocyte count were measured for up to 12 or 3 weeks after the start of the treatment for each of the cohorts. Figure 19 shows the median total number of all lymphocytes for each of the four cohorts up to 13 weeks after treatment. Figure 20 shows the median total number of CD4+ T-lymphocytes for each of the four cohorts up to 13 weeks after treatment. Figure 21 shows the median total number of CD8+ T-lymphocytes for each of the four cohorts up to 13 weeks after treatment. Figure 22 shows the median total number of TCR+ (CD4+ and CD8+) T-lymphocytes for each of the four cohorts up to 13 weeks after treatment. Figure 23 shows the median total number of CD2+ T-lymphocytes for each of the four cohorts up to 13 weeks after treatment.

### EXAMPLE 5

Based upon the pharmacokinetic and pharmacodynamic data obtained in Examples 3 and 4, an 8-day TRX4 dosing regimen was developed. In this example, each patient in a cohort of six patients received intravenous doses of 0.1 mg TRX4 on Day 1, 0.2 mg TRX4 on Day 2, 0.3 mg TRX4 on Day 3, and 0.5 mg TRX4 on each of Days 4 through 8. This regimen or protocol was entitled TRX4 Therapeutic Evaluation of Different Multi-Dose Regimens in Type 1 Diabetes Mellitus, or TTEDD.

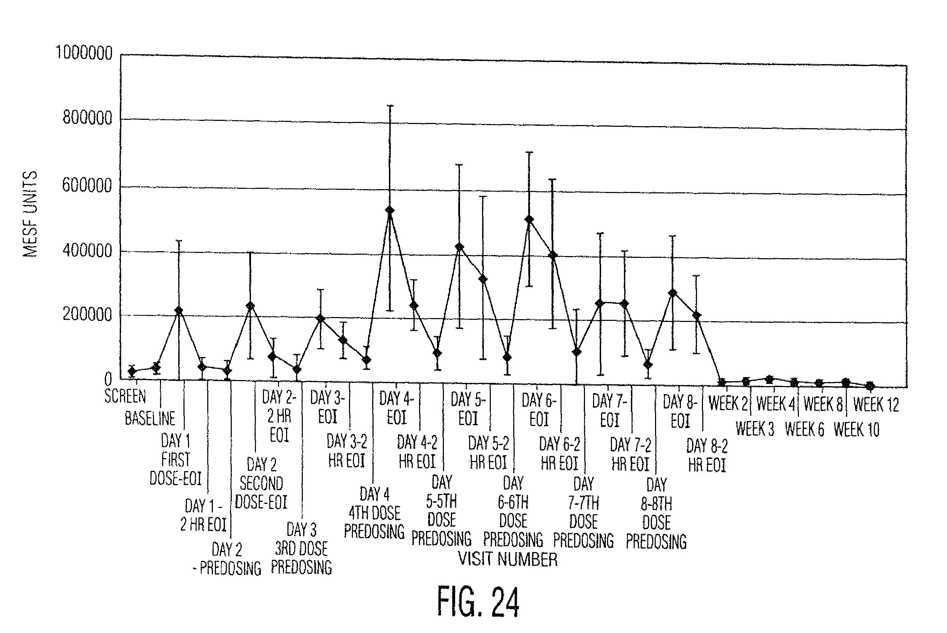

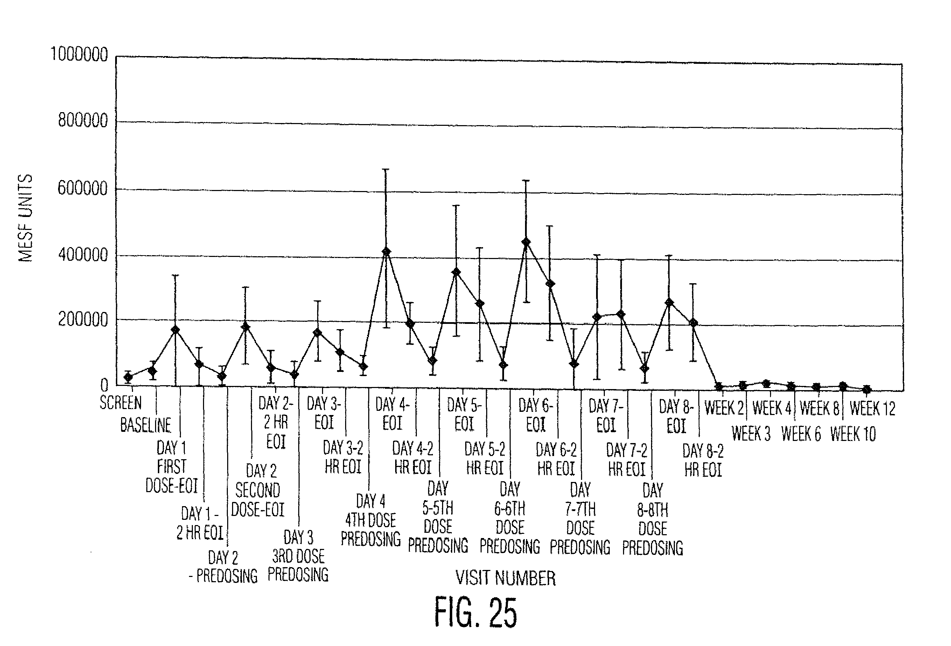

In this example, cell-bound TRX4 on CD4+ T cells and CD8+ T-cells was determined using anti-human IgG antibody reagents, and fluorescence intensity was quantified by using standard MESF units as described in Example 3. Results are shown graphically in Figures 24 and 25.

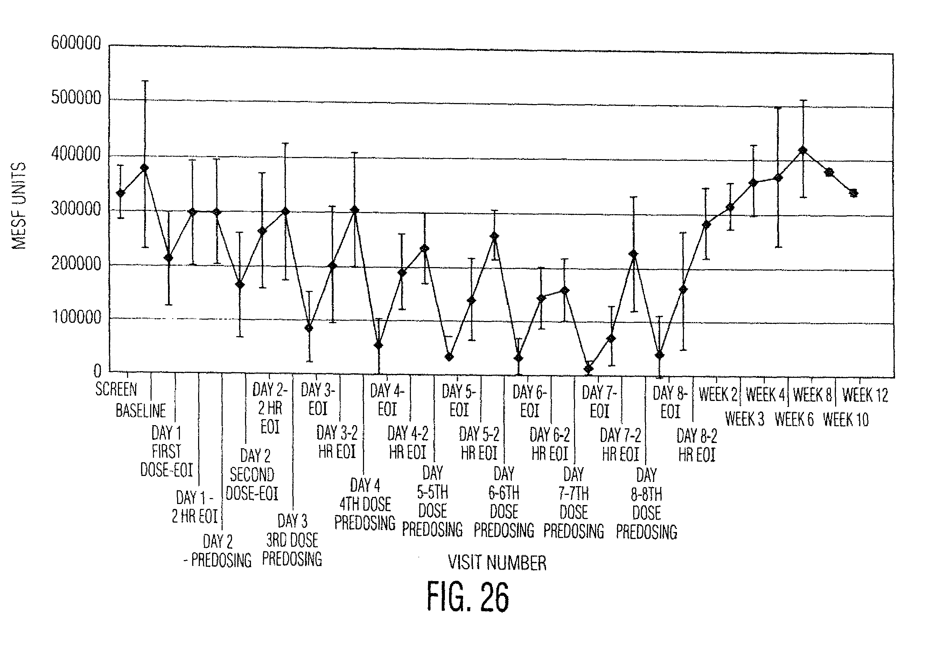

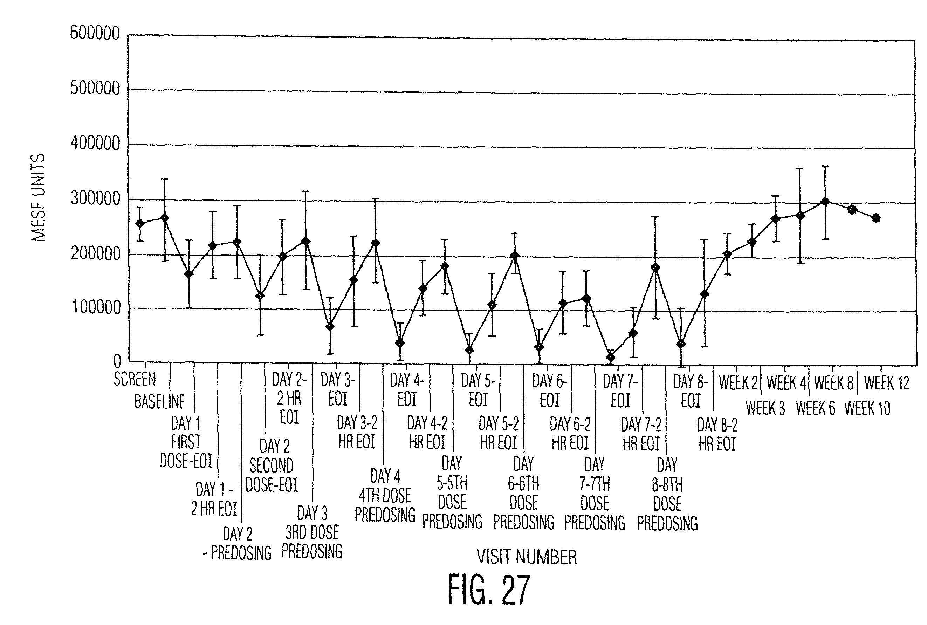

Free TRX4 CD3 binding sites (unoccupied by previously administered TRX4) on CD4+ T cells and CD8+ T-cells were detected by staining with biotinylated TRX4 as described in Example 3. The MESF of bound biotinylated TRX4 is directly proportional to the availability of free TRX4 binding sites. Results are shown graphically in Figures 26 and 27.

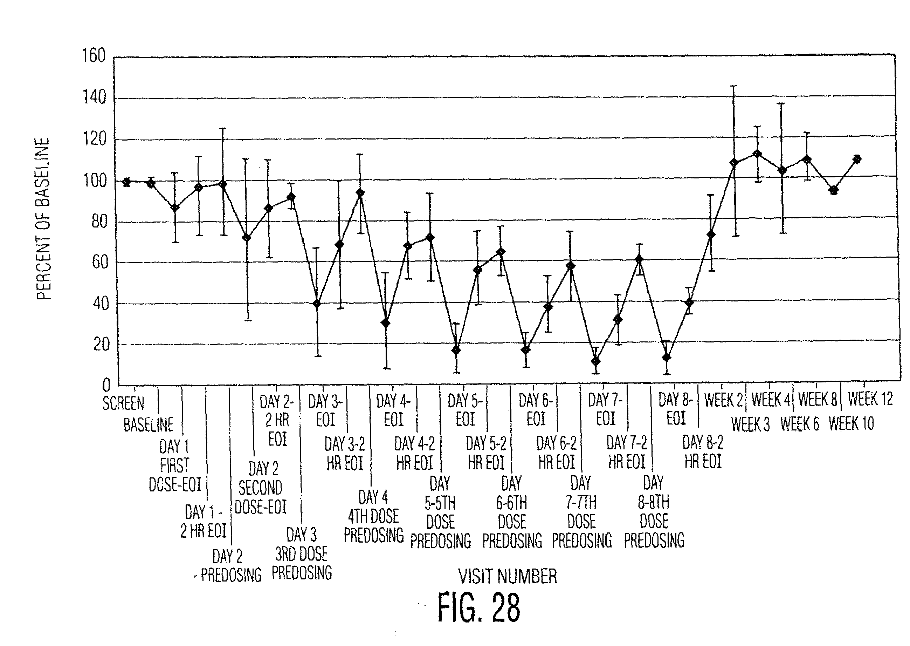

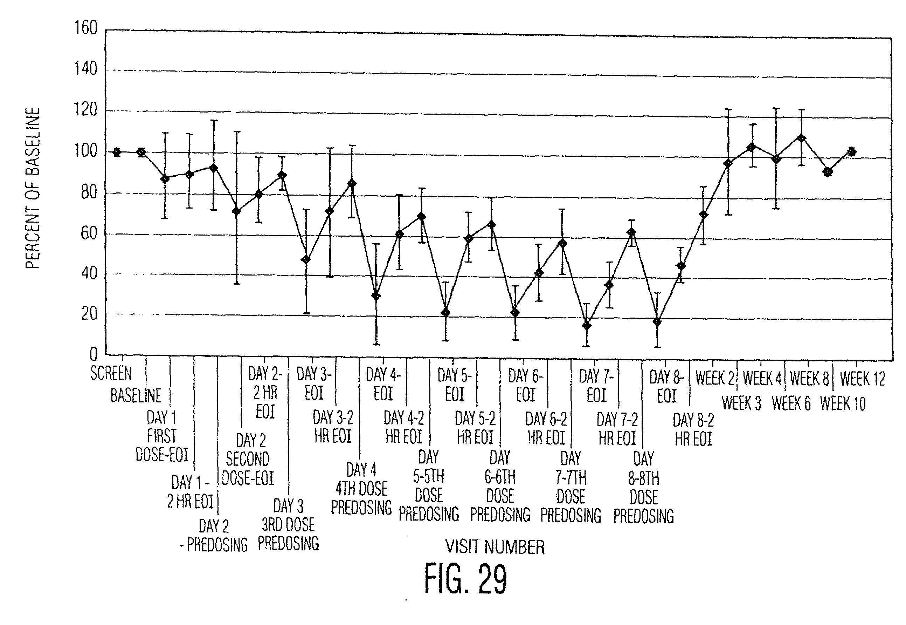

In order to evaluate the level of surface expression of the CD3/TCR complex and its modulation, αß TCR expression was determined for CD4+ T cells and CD8+ T-cells using the antibody BMA01 as described in Example 3. As noted hereinabove, binding of this antibody is not blocked by TRX4 bound to the CD3 surface molecule when TRX4 serum levels are below 1 µg/ml. The MESF of the anti-TCR αß antibody was used to quantify the number of CD3/TCR complexes present on T cells. Results are shown graphically in Figures 28 and 29 as percentages of the baseline value.

Absolute counts for lymphocytes and for each lymphocyte subset per liter were calculated based on CD markers as described in Example 3. Absolute counts and percentages were calculated for each parameter, and changes from baseline were determined for each post-baseline assessment.

### Detection of TRX4 Bound to CD4+ T cells and CD8+ T cells

The amounts of TRX4 bound to CD4+ T cells (Figure 24) and bound to CD8+ T cells (Figure 25) increased significantly above baseline within 2 hours after each infusion, and then decreased before the next day's infusion. The amounts of TRX4 bound to CD4+ T calls and CD8+ T cells prior to the infusions on Days 2 through 8, however, were at or above baseline. The amount of binding was greatest after the infusions on Days 4 and 6. When the infusions of TRX4 were stopped after Day 8, TRX4 binding levels had returned to baseline between Week 2 and Week 3.

### T Cell Receptor Analysis

The number of free CD3 sites on CD4+ T cells (Figure 26) and on CD8+ T cells (Figure 27) decreased after each infusion of TRX4. Almost complete saturation was achieved after the infusion on Day 7. When the infusions were stopped after Day 8, free CD3 sites on CD4+ T-cells returned to near baseline levels at about Week 5 (Figure 26) and free CD3 sites on CD8+ T cells returned to near baseline levels at about Week 4 (Figure 27).

CD3/TCR modulation on CD4+ T cells (Figure 28) and on CD8+ T cells (Figure 29) was observed at the end of each TRX4 infusion. Greater than 80% modulation on CD4+ T cells was observed after the infusions on each of Days 5 through 8 (Figure 28). Approximately 80% modulation on CD8+ T cells was observed after the infusions on each of Days 5 through 8 (Figure 29). When the infusions were stopped after Day 8, modulation of CD4+ T cells was observed until about Week 3 (Figure 28), and modulation of CD8+ T cells also was observed until about Week 3. (Figure 29).

### Lymphocyte Numbers

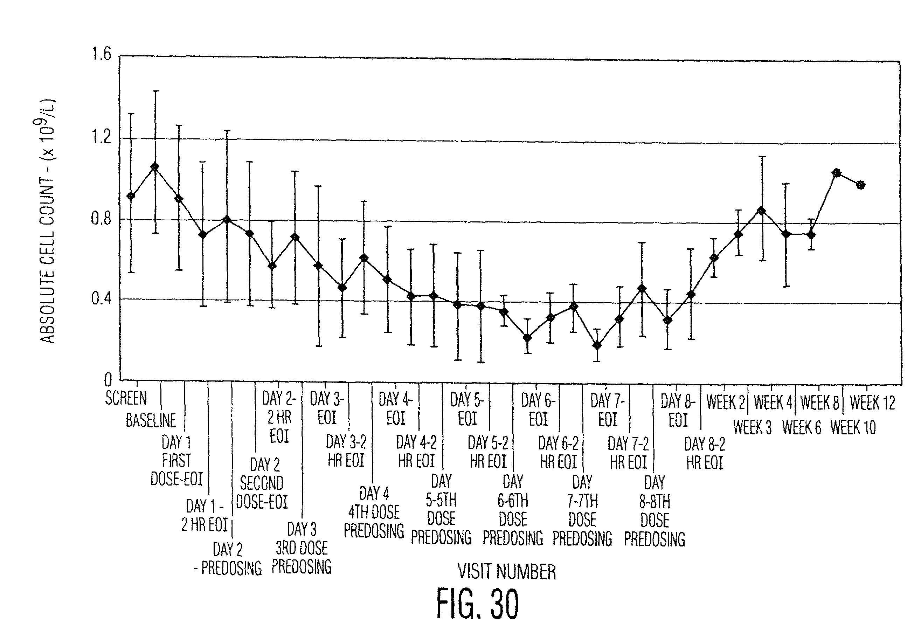

The CD4+ T cell count was reduced after the first infusion of TRX4 and remained below baseline levels during the 8 days of dosing (Figure 30). Levels were at about 20% of baseline after the TRX4 infusion on Day 7. When the infusions were stopped after Day 8, the CD4+ T cell count increased gradually, but did not return to baseline until approximately Week 10.

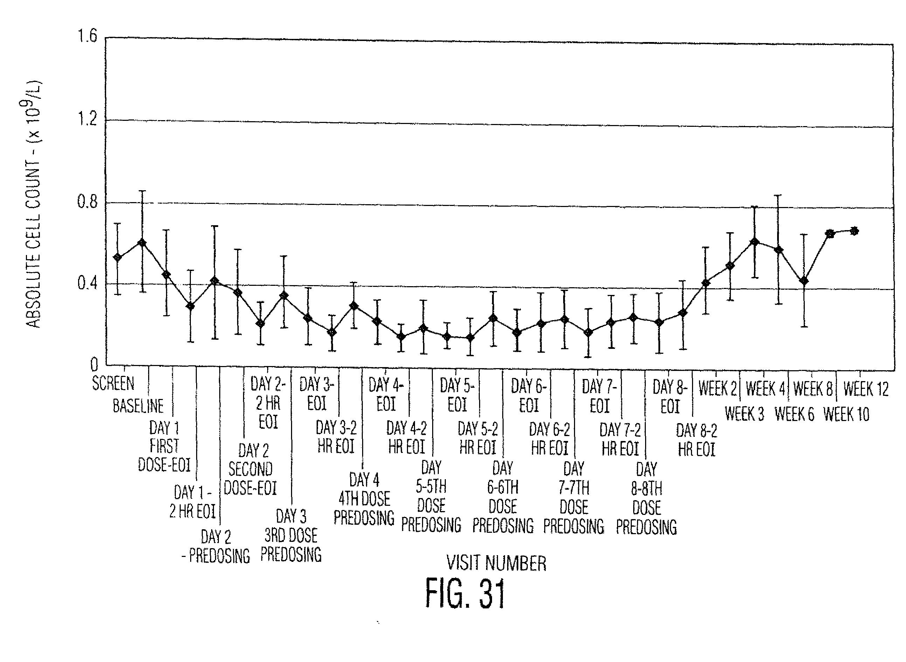

CD8+ T cell counts also were reduced after the first infusion of TRX4 and remained significantly below baseline levels throughout the 8 days of dosing (Figure 31). When the infusions were stopped after Day 8, the CD8+ T cell count increased, but did not reach baseline levels until about Week 6.

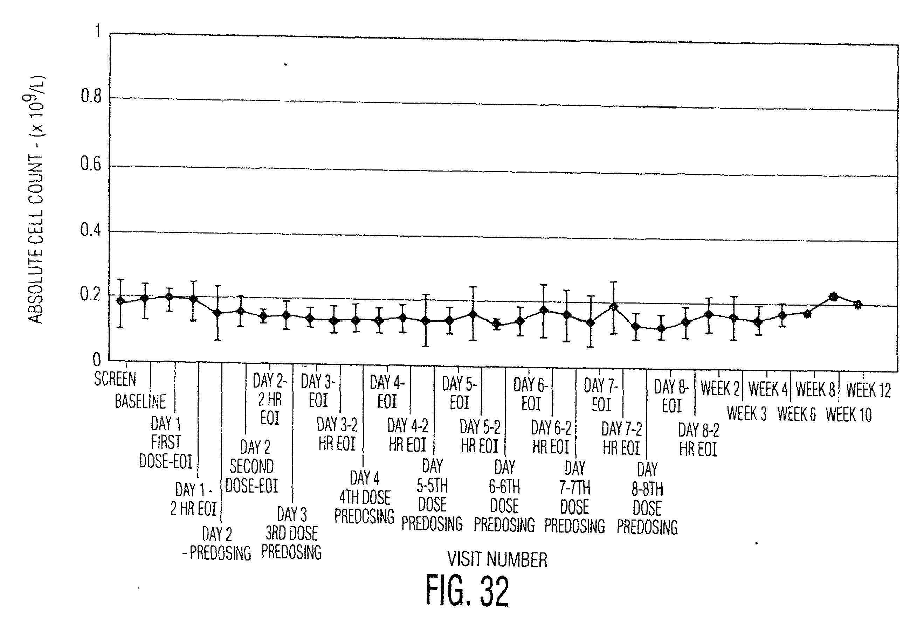

CD19+ B cell counts remained at or near baseline levels throughout the 8 day dosing regimen, and up to and through Week 12. (Figure 32).

The pharmacodynamic parameters observed in the 8 day TRX4 dosing regimen described herein were compared with other TRX4 dosing regimens, namely (i) the European Union Phase II trial in which 35 new onset Type I Diabetes patients were given infusions of 8 mg of TRX4 on six consecutive days (Keymeulen, 2005), and (ii) a study in which each patient in a cohort of four patients suffering from psoriasis was given a single intravenous infusion of 1 mg of TRX4. This study is described further in Example 6 hereinbelow. A placebo group of 40 patients who received 0.9% saline solution was used as a control.

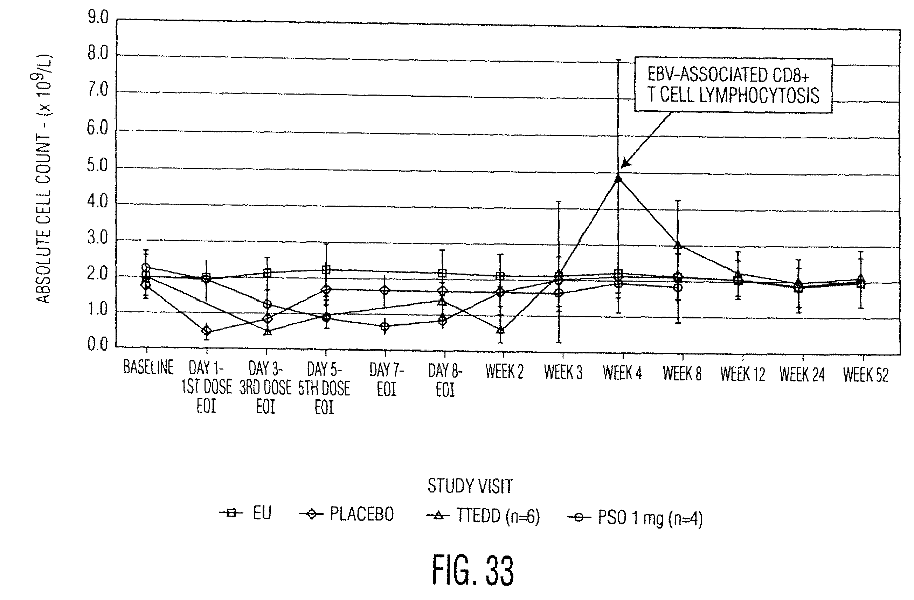

Figure 33 shows the average total number of lymphocytes in the six patients treated in this example, as compared to the patients treated with the humanized aglycosylated anti-CD3 antibody in the European Union Phase II trial (EU) (Keymeulen, 2005), and the four psoriasis patients given 1 mg TRX4 (PSO). As shown in Figure 33, the total numbers of lymphocytes in the six patients treated in this example and the psoriasis patients approximated each other over a period of 10 weeks, and approximated the total number of lymphocytes in the patients of the European trial for approximately 3 weeks. The patients in the European trial then had a significant rise in the number of lymphocytes, which was due to Epstein-Barr Virus (EBV) - associated CD8+ T cell lymphocytosis.

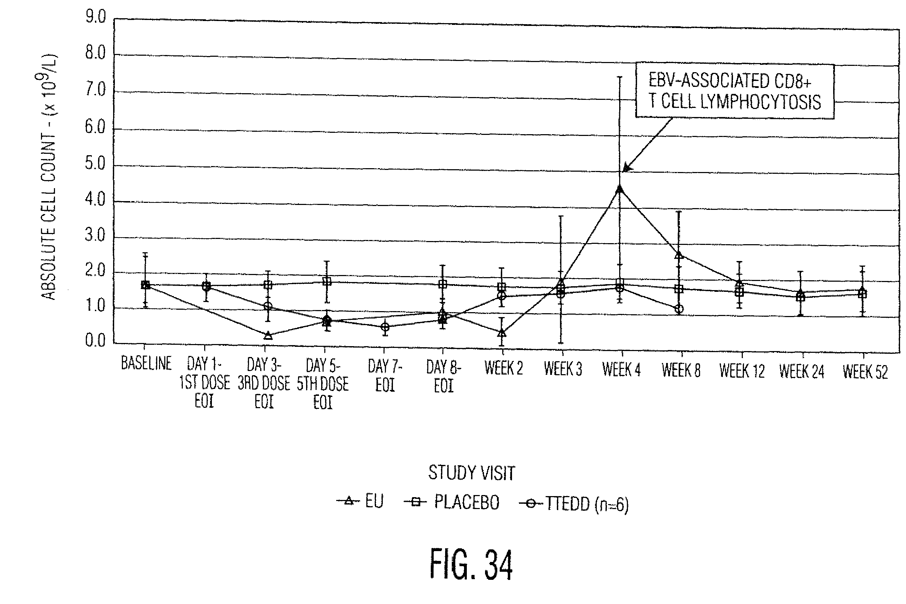

Figure 34 shows the number of CD2+ T lymphocytes in the six patients treated in this example, as well as those in the Type 1 diabetes patients treated in the European study. The numbers of CD2+ T lymphocytes in these groups approximated each other until Week 3, after which occurred a significant rise in the number of CD2+ T lymphocytes in the patients treated in the European study. This again was due to EBV-associated CD8+ T cell lymphocytosis, because the CD2+ T lymphocytes also are CD8+.

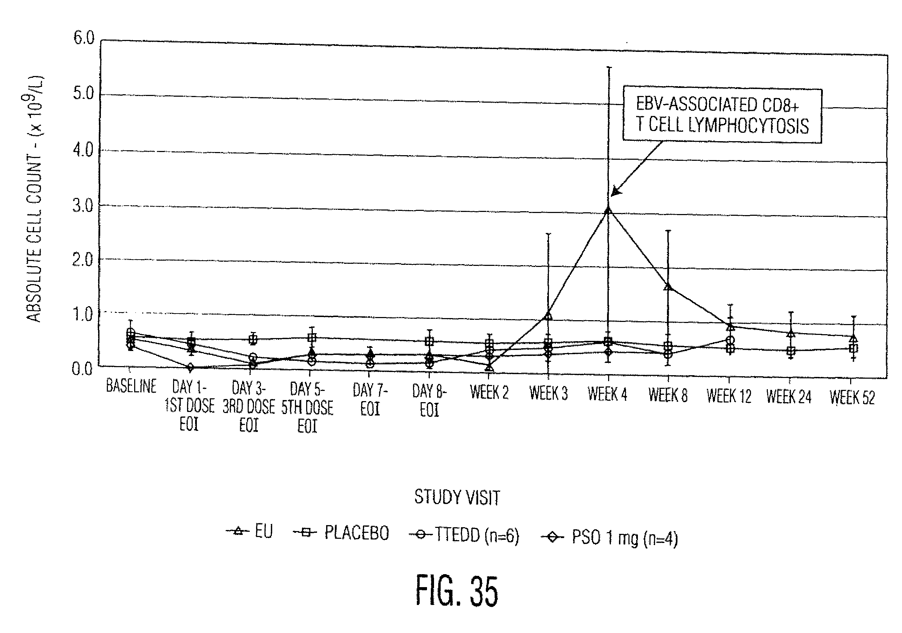

Figure 35 shows the total number of CD8+ T lymphocytes in the six patients treated in this example, as well as the Type I diabetes patients treated in the European Phase II trial, and the four psoriasis patients who were given 1 mg TRX4. The numbers of CD8+ T lymphocytes in the six patients treated in this example and the psoriasis patients approximated each other over a period of 10 weeks, while the numbers of CD8+ T lymphocytes of the six patients treated in this example and the Type 1 diabetes patients treated in the European Phase II trial approximated each other for a period of about 2 weeks, after which there was a significant rise in the number of CD8+T lymphocytes in the Type 1 diabetes patients treated in the European Phase II trial. This rise in the number of CD8+ T lymphocytes was due to EBV-associated CD8+ T cell lymphocytosis.

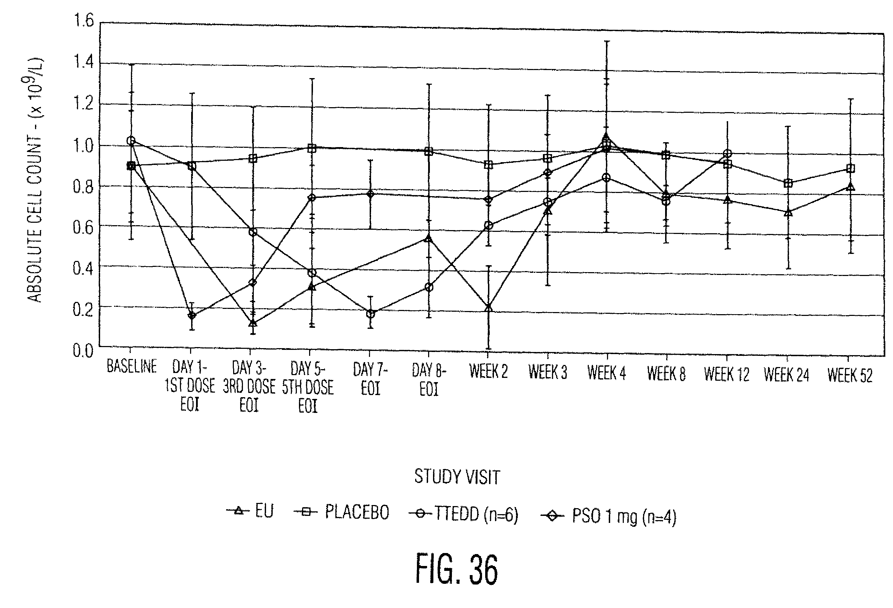

Figure 36 shows the CD4+ T lymphocyte counts for the six patients treated in this example, as well as the Type I diabetes patients treated in the European Phase II trial, and the four psoriasis patients who received 1 mg TRX4. The six patients treated in this example, the type 1 diabetes patients treated in the European Phase II trial, and the four psoriasis patients who received 1 mg TRX4 had similar decreases and increases in the number of CD4+ T lymphocytes over a period of 10 weeks.

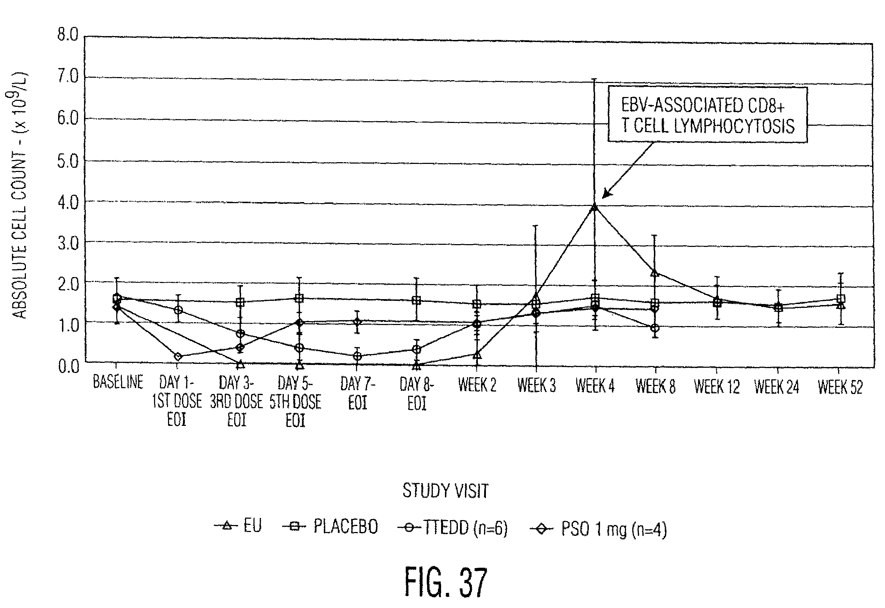

Figure 37 shows the TCR+ T lymphocyte counts for the six patients treated in this example, the Type 1 diabetes patients treated in the European Phase II trial, and the four psoriasis patients who received 1 mg of TRX4. Such TCR+ T lymphocytes are CD4+ and CD8+. The numbers of TCR+ T lymphocytes in the six patients treated in this example and the four psoriasis patients approximated each other over a period of 10 weeks. Similar changes in the numbers of TCR+ T lymphocytes were observed in the six patients treated in this study and the Type 1 diabetes patients treated in the European Phase II trial over a period of about 2 weeks, after which there was a significant rise in the number of TCR+ T lymphocytes in the Type 1 diabetes patients treated in the European Phase II trial. This was due to EBV-associated CD8+ and CD4+T cell lymphocytosis.

As shown in Figures 33 through 37, the pharmacodynamic parameters, as measured in terms of various lymphocyte counts, of the six patients treated in this example, were similar to those of the Type 1 diabetes patients treated in the Phase II European trial (Keymeulen, 2005), and the four psoriasis patients who received 1 mg of TRX4.

As has been noted previously (Keymeulen, 2005), the Type 1 diabetes patients in the European Phase II trial who received the humanized aglycosylated anti-CD3 antibody TRX4 had better maintenance of residual beta cell function than those in the placebo group. Insulin doses also increased in the placebo group but not in the patients treated with the CD3 antibody.

As will be explained in further detail in Example 6 hereinbelow, the four psoriasis patients who received an intravenous infusion of 1 mg of TRX4 showed a reduction in their psoriasis area severity index (PASI) scores at 8 weeks post-infusion.

Thus, the treatment regimen employing TRX4 as described in this example has pharmacodynamic parameters similar to other treatment regimens employing anti-CD3 antibodies, which have been effective in treating diabetes or psoriasis.

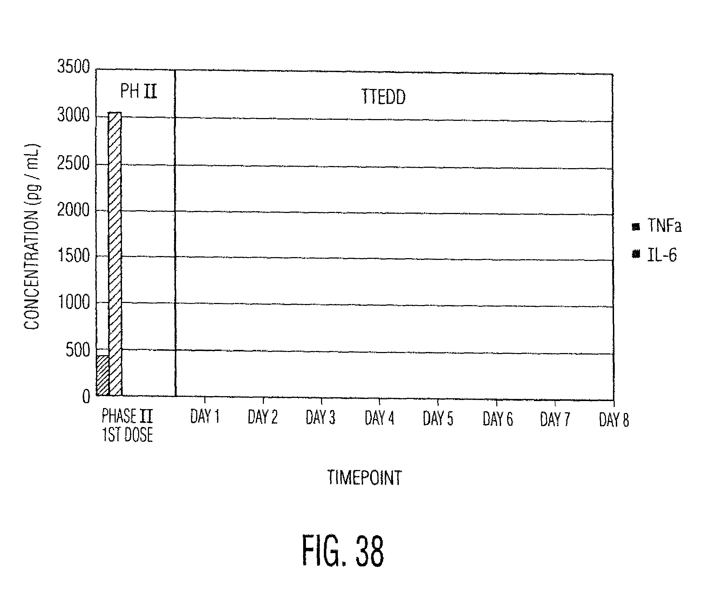

In addition, release of the cytokines TNF-α and Interleukin-6 (IL-6) was measured by an ELISA assay (R and D Systems, Minneapolis, MN) at one hour after the end of infusion on each of the eight days of the treatment regimen, and the mean cytokine release for each of TNF-α and IL-6 in pg/ml was determined. The mean cytokine release concentrations for each of Days 1 through 8 were compared to the mean cytokine release concentrations for the patients in the Phase II European trial (Keymeulen, 2005) at one hour after the end of infusion of their first dose of 8 mg of TRX4. The results are shown graphically in Figure 38. As shown in Figure 38, the dosing regimen of 0.1 mg TRX4 on Day 1, 0.2 mg TRX4 on Day 2, 0.3 mg TRX4 on Day 3, and 0.5 mg TRX4 on Days 4 through 8 provided a significant reduction in the release of the cytokines TNF-α and IL-6, as compared to the Phase II European trial.

### EXAMPLE 6

Four human patients, referred to herein as Cohort I, each had received a physician's assessment of at least moderate psoriasis with at least 10% of the body surface area affected, and were eligible for or had systemic therapy. Each patient of Cohort 1 was given 1 mg of TRX4 by intravenous infusion over a period of one hour. None of the patients of Cohort 1 had received any systemic agents for psoriasis treatment or any potent immunosuppressive agents within four weeks prior to receiving TRX4. During the eight week period following administration of TRX4, none of the patients of Cohort 1 received any topical or systemic treatment for psoriasis.

Immediately after the administration of TRX4, it was observed that the absolute lymphocyte counts for all patients of Cohort 1 had decreased. The decrease was observed just after the completion of the one-hour infusion of TRX4, and was most prominent between Days 1 and 2, and an increase of the lymphocyte counts began on Day 3. The lymphocyte counts returned to baseline by Day 6.

Modulation of the CD3/TCR complex, as detected by staining with an antibody to the TCR, was observed most profoundly immediately after the administration of TRX4, with full recovery of the TCR complex on the cell surface within 6 days after dosing. Serum levels of TRX4 were detected in the blood immediately after completion of the administration of TRX4, and for up to one hour after administration.

Baseline PASI scores were measured for each of the patients in Cohort 1 at one day (Day-1) before receiving TRX4. PASI scores also were measured for each of the patients in Cohort 1 during Week 8 after receiving TRX4.

The PASI scores for each of the four patients of Cohort 1, who received a 1 mg intravenous infusion of TRX4, at baseline (Day-1) and during Week 8 after administration of TRX4, are shown in Table 3 hereinbelow.

**Table 3**

| | PASI Score | |
|---|---|---|
| Patient | Day-1 | Week 8 |
| 1 | 12.2 | 6.6 |
| 2 | 27.0 | 16.9 |
| 3 | 15.8 | 7.8 |
| 4 | 16.4 | 8.5 |

In addition, five human patients, hereinafter referred to as "Cohort 2", were given 2 mg of TRX4 antibody, and seven human patients, hereinafter referred to as "Cohort 3", were given 4 mg of TRX4 antibody.

As with Cohort 1, each of the patients of Cohort 2 and Cohort 3 was given TRX4 by intravenous infusion over a period of one hour.

As with Cohort 1, each of the patients of Cohort 2 and Cohort 3 had received a physician's assessment of at least moderate psoriasis with at least 10% of body surface area affected, and were eligible for or had systemic therapy.

None of the patients of Cohort 2 or Cohort 3 had received any systemic agents for psoriasis treatment or any potent immunosuppressive agents within four weeks prior to receiving TRX4.

Baseline PASI scores were measured for each of the patients in Cohort 2 and Cohort 3 at one day (Day-1) before receiving TRX4. PASI scores also were measured for each of the patients in Cohort 2 and Cohort 3 during Week 8 after receiving TRX4.

The PASI scores of Patients 1 through 5 of Cohort 2 are given in Table 4 hereinbelow. Patient 5 of Cohort 2, at 7 weeks after receiving TRX4, started to receive a daily topical application of a topical corticosteroid cream including 0.05% halobetasol propionate, an agent used for the treatment of psoriasis. The remaining patients of Cohort 2 did not receive any topical or systemic treatment for psoriasis in the 8 weeks after receiving TRX4.

The PASI scores for each of the five patients of Cohort 2, who received a 2 mg intravenous infusion of TRX4, at baseline (Day-1) and during Week 8 after administration of TRX4, were as follows:

**Table 4**

| | PASI Score | |
|---|---|---|
| Patient | Day-1 | Week 8 |
| 1 | 13.4 | 10.8 |
| 2 | 13.8 | 14.6 |
| 3 | 12.8 | 11.1 |
| 4 | 12.5 | 4.9 |
| 5 | 10.7 | 8.9 |

The PASI scores of Patients 1 through 7 of Cohort 3 are given in Table 5 hereinbelow. Except for Patient 7, none of the patients of Cohort 3 received any topical or systemic treatment for psoriasis in the 8 weeks after receiving TRX4. Patient 7 began Ultraviolet B light therapy for psoriasis one week after receiving TRX4.

**Table 5**

| | PASI Score | |
|---|---|---|
| Patient | Day-1 | Week 8 |
| 1 | 14.2 | 10.8 |
| 2 | 17.6 | 14.7 |
| 3 | 14.5 | 9.4 |
| 4 | 12.9 | 15.0 |
| 5 | 20.1 | 17.8 |
| 6 | 23.1 | 17.6 |
| 7 | 25.8 | 17.8 |

The disclosures of all patents, publications (including published patent applications), depository accession numbers, and database accession numbers are incorporated herein by reference to the same extent as if each patent, publication, depository accession number, and database accession number were specifically and individually incorporated by reference.

It is to be understood, however, that the scope of the present invention is not to be limited to the specific embodiments described above. The invention may be practiced other than as particularly described and still be within the scope of the accompanying claims.

Further preferred embodiments are described below.

A further preferred embodiment is directed to a method of treating an automimmune disease, comprising:
administering to an animal an anti-CD3 antibody or anti-CD3 antibody fragment selected from the group consisting of anti-CD3 antibodies and fragments thereof which do not bind or have reduced binding to the Fc (gamma) receptors, over a course of treatment, wherein over the course of treatment, said anti-CD3 antibody or anti-CD3 antibody fragment is administered in an amount wherein the total amount does not exceed 300 µg/kg when administered intravenously, and when administered other than intravenously the total amount administered does not exceed the bioequivalent of intravenous administration of 300 µg/kg;
   ● wherein preferably said autoimmune disease is diabetes;
   ● wherein preferably said autoimmune disease is psoriasis;
   ● wherein preferably said antibody or fragment is administered in a therapeutically effective amount in which the total amount does not exceed 175 µg/kg when administered intravenously and when administered other than intravenously the total amount does not exceed the amount bioequivalent to intravenous administration of 175 µg/kg;
      o wherein further preferably said antibody or fragment is administered in a total amount which does not exceed 150 µg/kg when administered intravenously and when administered other than intravenously the total amount does not exceed the amount bioequivalent to intravenous administration of 150 µg/kg;
         ■ wherein further preferably said antibody or fragment thereof is administered in a total amount which does not exceed 50 µg/kg when administered intravenously and when administered other than intravenously the total amount does not exceed the amount bioequivalent to intravenous administration of 50 µg/kg;
            ● wherein further preferably said antibody or fragment is administered in a total amount which is at least 8 µg/kg when administered intravenously and when administered other than intravenously the total amount is at least an amount bioequivalent to intravenous administration of 8 µg/kg;
   ● wherein preferably said anti-CD3 antibody or anti-CD3 antibody fragment is administered in an amount which is not less than 0.1 µg/kg per day when administered intravenously and when administered other than intravenously the amount is not less than the amount bioequivalent to intravenous administration thereof of 0.1 µg/kg per day;
   ● wherein preferably said anti-CD3 antibody or anti-CD3 antibody fragment is administered in an amount which does not exceed 30 µg/kg per day when administered intravenously and when administered other than intravenously the amount does not exceed the amount bioequivalent to intravenous administration thereof of 30 µg/kg per day;
      o wherein further preferably said anti-CD3 antibody or anti-CD3 antibody fragment is administered in an amount which does not exceed 25 µg/kg per day when administered intravenously and when administered other than intravenously the amount does not exceed the amount bioequivalent to intravenous administration thereof of 25 µg/kg per day;
         ■ wherein further preferably said anti-CD3 antibody or anti-CD3 antibody fragment is administered in an amount which does not exceed 20 µg/kg per day when administered intravenously and when administered other than intravenously the amount does not exceed the amount bioequivalent to intravenous administration thereof of 20 µg/kg per day;
            ● wherein further preferably said anti-CD3 antibody or anti-CD3 antibody fragment is administered in an amount which does not exceed 15 µg/kg per day when administered intravenously and when administered other than intravenously the amount does not exceed the amount bioequivalent to intravenous administration thereof of 15 µg/kg per day;
               o wherein further preferably said anti-CD3 antibody or anti-CD3 antibody fragment is administered in an amount which does not exceed 10 µg/kg per day when administered intravenously and when administered other than intravenously the amount does not exceed the amount bioequivalent to intravenous administration thereof of 10 µg/kg per day;
   ● wherein preferably said course of treatment does not exceed 10 days;
      o wherein further preferably said course of treatment does not exceed 8 days;
         ■ wherein further preferably said course of treatment does not exceed 6 days;
   ● wherein preferably said antibody or fragment is administered intravenously;
   ● wherein preferably said animal is a mammal;
      o wherein further preferably said mammal is a primate;
         ■ wherein further preferably said primate is a human;

A further preferred embodiment is directed to a method of effecting T-cell receptor modulation in an animal comprising:
administering to an animal an anti-CD3 antibody or anti-CD3 antibody fragment selected from the group consisting of anti-CD3 antibodies and fragments thereof which do not bind or have reduced binding to the Fc (gamma) receptors, said anti-CD3 antibody or anti-CD3 antibody fragment being administered in an amount effective to effect T-cell receptor modulation in the animal;
   ● wherein preferably said antibody or fragment is administered intravenously;
   ● wherein preferably said animal is a mammal;
      o wherein further preferably said mammal is a primate;
         ■ wherein further preferably said primate is a human;

A further preferred embodiment is directed to a product comprising:
an anti-CD3 antibody, o anti-CD3 antibody fragment, which does not bind or has reduced binding to the Fc (gamma) receptors;
an acceptable pharmaceutical carrier; and
instructions to administer said antibody, or fragment, for the treatment of diabetes over a course of treatment wherein, during said course of treatment, the total amount of said antibody, or fragment, is administered in an amount which does not exceed 300 µg/kg when administered intravenously.

## Claims

1. Use of an anti-CD3 antibody or anti-CD3 antibody fragment selected from the group consisting of anti-CD3 IgG 1 or IgG3 antibodies and fragments thereof which do not bind or have reduced binding to the Fc (gamma) receptors, for the manufacture of a pharmaceutical preparation for treating an autoimmune disease wherein over the course of a treatment, said anti-CD3 antibody, or a bioequivalent amount of anti-CD3 antibody fragment, is to be administered in an amount wherein the total amount does not exceed 300 µg/kg when to be administered intravenously, and when to be administered other than intravenously the total amount administered does not exceed the bioequivalent of intravenous administration of 300 µg/kg.

2. The use of Claim 1 wherein said autoimmune disease is diabetes.

3. The use of Claim 1 wherein said autoimmune disease is psoriasis.

4. The use of Claim 1 wherein said antibody, or a bioequivalent amount of fragment, is to be administered in a therapeutically effective amount in which the total amount does not exceed 175 µg/kg when to be administered intravenously and when to be administered other than intravenously the total amount does not exceed the amount bioequivalent to intravenous administration of 175 µg/kg.

5. The use of Claim 4 wherein said antibody, or a bioequivalent amount of fragment, is to be administered in a total amount which does not exceed 150 µg/kg when to be administered intravenously and when to be administered other than intravenously the total amount does not exceed the amount bioequivalent to intravenous administration of 150 µg/kg.

6. The use of Claim 5 wherein said antibody, or a bioequivalent amount of fragment, thereof is to be administered in a total amount which does not exceed 50 µg/kg when to be administered intravenously and when to be administered other than intravenously the total amount does not exceed the amount bioequivalent to intravenous administration of 50 µg/kg.

7. The use of Claim 6 wherein said antibody, or a bioequivalent amount of fragment, is to be administered in a total amount which is at least 8 µg/kg when to be administered intravenously and when to be administered other than intravenously the total amount is at least an amount bioequivalent to intravenous administration of 8 µg/kg.

8. The use of Claim 1 wherein said anti-CD3 antibody, or a bioequivalent amount of anti-CD3 antibody fragment, is to be administered in an amount which is not less than 0.1 µg/kg per day when to be administered intravenously and when to be administered other than intravenously the amount is not less than the amount bioequivalent to intravenous administration thereof of 0.1 µg/kg per day.

9. The use of Claim 1 wherein said anti-CD3 antibody, or a bioequivalent amount of anti-CD3 antibody fragment, is to be administered in an amount which does not exceed 30 µg/kg per day when to be administered intravenously and when to be administered other than intravenously the amount does not exceed the amount bioequivalent to intravenous administration thereof of 30 µg/kg per day.

10. The use of Claim 9 wherein said anti-CD3 antibody, or a bioequivalent amount of anti-CD3 antibody fragment, is to be administered in an amount which does not exceed 25 µg/kg per day when to be administered intravenously and when to be administered other than intravenously the amount does not exceed the amount bioequivalent to intravenous administration thereof of 25 µg/kg per day.

11. The use of Claim 10 wherein said anti-CD3 antibody, or a bioequivalent amount of anti-CD3 antibody fragment is to be administered in an amount which does not exceed 20 µg/kg per day when to be administered intravenously and when to be administered other than intravenously the amount does not exceed the amount bioequivalent to intravenous administration thereof of 20 µg/kg per day.

12. The use of Claim I wherein said anti-CD3 antibody, or a bioequivalent amount of anti-CD3 antibody fragment, is to be administered in an amount which does not exceed 15 µg/kg per day when to be administered intravenously and when to be administered other than intravenously the amount does not exceed the amount bioequivalent to intravenous administration thereof of 15 µg/kg per day.

13. The use of Claim 12 wherein said anti-CD3 antibody, or a bioequivalent amount of anti-CD3 antibody fragment, is to be administered in an amount which does not exceed 10 µg/kg per day when to be administered intravenously and when to be administered other than intravenously the amount does not exceed the amount bioequivalent to intravenous administration thereof of 10 µg/kg per day.

14. The use of Claim 1 wherein said course of treatment does not exceed 10 days.

15. The use of Claim 14 wherein said course of treatment does not exceed 8 days.

16. The use of Claim 15 wherein said course of treatment does not exceed 6 days.

17. The use of Claim 1 wherein said antibody or fragment is to be administered intravenously.

18. Use of an anti-CD3 antibody or anti-CD3 antibody fragment selected from the group consisting of anti-CD3 IgG1 or IgG3 antibodies and fragments thereof which do not bind or have reduced binding to the Fc (gamma) receptors, for the manufacture of a pharmaceutical preparation for effecting T-cell receptor modulation.

19. The use of Claim 18 wherein said antibody or fragment is to be administered intravenously.

20. A product, comprising:
an anti-CD3 IgG1 or IgG3 antibody, or anti-CD3 IgG1 or IgG3 antibody fragment, which does not bind or has reduced binding to the Fc (gamma) receptors;
an acceptable pharmaceutical carrier; and
instructions to administer said antibody, or fragment, for the treatment of diabetes over a course of treatment wherein, during said course of treatment, the total amount of said antibody, or a bioequivalent amount of fragment, is administered in an amount which does not exceed 300 µg/kg when administered intravenously.

21. The use of any of claims 1 to 17 wherein the anti-CD3 antibody or anti-CD3 antibody fragment, when to be administered other than intravenously, is to be administered by methods such as intra-arterial, intraperitoneal, intramuscular or subcutaneous administration.

22. The use of any of claims 1 to 19 wherein the dose is increased on each succeeding day of treatment until a preselected maximum daily dosage is reached.

23. The use of Claim 1 wherein said anti-CD3 antibody or anti-CD3 antibody fragment is administered in more than one course of treatment.

24. The use of Claim 22 wherein the anti-CD3 antibody or anti-CD3 antibody fragment is administered in an amount of about 1.4 µg/kg on Day 1 of the treatment, in an amount of about 2.8 µg/kg on Day 2 of the treatment, and in an amount of about 4.3 µg/kg on Day 3 of the treatment and from Day 4 to Day 8 of the treatment in an amount of about 7.1 µg/kg on each of such days of the treatment.

25. The use of Claim 1 wherein said anti-CD3 antibody is the TRX4 antibody.

26. The use of Claim 25 wherein said TRX4 antibody is administered over a course of treatment of 8 days as intravenous doses of 0.1 mg TRX4 on Day 1, 0.2 mg TRX4 on Day 2, 0.3 mg TRX4 on Day 3, and 0.5 mg TRX4 on each of Days 4 through 8.


SEQUENCE LISTING

<110> Tolerrx Inc.

<120> Administration of CD3 Antibodies in the Treatment of Autoimmune
      Diseases

<130> E/49645EPWT1/AW/js

<140> PCT/US2007/013232
<141> 2007-06-05

<150> US2006/0447628
<151> 2006-06-06

<160> 4

<170> PatentIn version 3.3

<210> 1
<211> 708
<212> DNA
<213> Artificial

<220>
<223> DNA encoding chimeric human/rat light chain of CD3 antibody

<400> 1
atgggatgga gctgtatcat cctcttcttg gtagcaacag ctacaggtgt ccactccgac      60

atccagctga ctcagcccaa ctctgtgtct acgtctctag gaagcacagt caagctgtct     120

tgcacactca gctctggtaa catagaaaac aactatgtgc actggtacca gctatatgag     180

ggaagatctc ccaccactat gatttatgat gatgataaga gaccggatgg tgtccctgac     240

aggttctctg gctccattga caggtcttcc aactcagcct tcctgacaat ccataatgtg     300

gcaattgaag atgaagctat ctacttctgt cattcttatg ttagtagttt taatgttttc     360

ggcggtggaa caaagctcac tgtccttcga cagcccaagg ctgccccctc ggtcactctg     420

ttcccgccct cctctgagga gcttcaagcc aacaaggcca cactggtgtg tctcataagt     480

gacttctacc cgggagccgt gacagtggcc tggaaggcag atagcagccc cgtcaaggcg     540

ggagtggaga ccaccacacc ctccaaacaa agcaacaaca agtacgcggc cagcagctac     600

ctgagcctga cgcctgagca gtggaagtcc cacagaagct acagctgcca ggtcacgcat     660

gaagggagca ccgtggagaa gacagtggcc cctacagaat gttcatag                  708

<210> 2
<211> 235
<212> PRT
<213> Artificial

<220>
<223> Chimeric human/rat light chain of monoclonal CD3 antibody

<400> 2

Met Gly Trp Ser Cys Ile Ile Leu Phe Leu Val Ala Thr Ala Thr Gly
1               5                   10                  15

Val His Ser Asp Ile Gln Leu Thr Gln Pro Asn Ser Val Ser Thr Ser
            20                  25                  30

Leu Gly Ser Thr Val Lys Leu Ser Cys Thr Leu Ser Ser Gly Asn Ile
        35                  40                  45

Glu Asn Asn Tyr Val His Trp Tyr Gln Leu Tyr Glu Gly Arg Ser Pro
    50                  55                  60

Thr Thr Met Ile Tyr Asp Asp Asp Lys Arg Pro Asp Gly Val Pro Asp
65                  70                  75                  80

Arg Phe Ser Gly Ser Ile Asp Arg Ser Ser Asn Ser Ala Phe Leu Thr
                85                  90                  95

Ile His Asn Val Ala Ile Glu Asp Glu Ala Ile Tyr Phe Cys His Ser
            100                 105                 110

Tyr Val Ser Ser Phe Asn Val Phe Gly Gly Gly Thr Lys Leu Thr Val
        115                 120                 125

Leu Arg Gln Pro Lys Ala Ala Pro Ser Val Thr Leu Phe Pro Pro Ser
    130                 135                 140

Ser Glu Glu Leu Gln Ala Asn Lys Ala Thr Leu Val Cys Leu Ile Ser
145                 150                 155                 160

Asp Phe Tyr Pro Gly Ala Val Thr Val Ala Trp Lys Ala Asp Ser Ser
                165                 170                 175

Pro Val Lys Ala Gly Val Glu Thr Thr Thr Pro Ser Lys Gln Ser Asn
            180                 185                 190

Asn Lys Tyr Ala Ala Ser Ser Tyr Leu Ser Leu Thr Pro Glu Gln Trp
        195                 200                 205

Lys Ser His Arg Ser Tyr Ser Cys Gln Val Thr His Glu Gly Ser Thr
    210                 215                 220

Val Glu Lys Thr Val Ala Pro Thr Glu Cys Ser
225                 230                 235

<210>  3
<211>  1407
<212>  DNA
<213>  Artificial

<220>
<223>  DNA encoding humanized heavy chain of CD3 antibody

<400>  3
atgggatgga gctgtatcat cctcttcttg gtagcaacag ctacaggtgt ccactccgag      60

gtccaactgc tggagtctgg gggcggttta gtgcagcctg gagggtccct gagactctcc     120

tgtgcagcct caggattcac tttcagtagc tttccaatgg cctgggtccg ccaggctcca     180

gggaagggtc tggagtgggt ctcaaccatt agtaccagtg gtggtagaac ttactatcga     240

gactccgtga agggccgatt cactatctcc agagataata gcaaaaatac cctatacctg     300

caaatgaata gtctgagggc tgaggacacg gccgtctatt actgtgcaaa atttcggcag     360

tacagtggtg gctttgatta ctggggccaa gggaccctgg tcaccgtctc ctcagcctcc     420

accaagggcc catcggtctt ccccctggca ccctcctcca agagcacctc tgggggcaca     480

gcggccctgg gctgcctggt caaggactac ttccccgaac cggtgacggt gtcgtggaac     540

tcaggcgccc tgaccagcgg cgtgcacacc ttcccggctg tcctacagtc ctcaggactc     600

tactccctca gcagcgtggt gaccgtgccc tccagcagct tgggcaccca gacctacatc     660

tgcaacgtga atcacaagcc cagcaacacc aaggtggaca agaaagttga gcccaaatct     720

tgtgacaaaa ctcacacatg cccaccgtgc ccagcacctg aactcctggg gggaccgtca     780

gtcttcctct tccccccaaa acccaaggac accctcatga tctcccggac ccctgaggtc     840

acatgcgtgg tggtggacgt gagccacgaa gaccctgagg tcaagttcaa ctggtacgtg     900

gacggcgtgg aggtgcataa tgccaagaca aagccgcggg aggagcagta cgccagcacg     960

taccgtgtgg tcagcgtcct caccgtcctg caccaggact ggctgaatgg caaggagtac    1020

aagtgcaagg tctccaacaa agccctccca gcccccatcg agaaaaccat ctccaaagcc    1080

aaagggcagc cccgagaacc acaggtgtac accctgcccc catcccggga tgagctgacc    1140

aagaaccagg tcagcctgac ctgcctggtc aaaggcttct atcccagcga catcgccgtg    1200

gagtgggaga gcaatgggca gccggagaac aactacaaga ccacgcctcc cgtgctggac    1260

tccgacggct ccttcttcct ctacagcaag ctcaccgtgg acaagagcag gtggcagcag    1320

gggaacgtct tctcatgctc cgtgatgcat gaggctctgc acaaccacta cacgcagaag    1380

agcctctccc tgtctccggg taaatga                                        1407

<210>  4
<211>  468
<212>  PRT
<213>  Artificial

<220>
<223>  Humanized heavy chain of monoclonal CD3 antibody

<400>  4

Met Gly Trp Ser Cys Ile Ile Leu Phe Leu Val Ala Thr Ala Thr Gly
1               5                   10                  15

Val His Ser Glu Val Gln Leu Leu Glu Ser Gly Gly Gly Leu Val Gln
            20                  25                  30

Pro Gly Gly Ser Leu Arg Leu Ser Cys Ala Ala Ser Gly Phe Thr Phe
        35                  40                  45

Ser Ser Phe Pro Met Ala Trp Val Arg Gln Ala Pro Gly Lys Gly Leu
    50                  55                  60

Glu Trp Val Ser Thr Ile Ser Thr Ser Gly Gly Arg Thr Tyr Tyr Arg
65                  70                  75                  80

Asp Ser Val Lys Gly Arg Phe Thr Ile Ser Arg Asp Asn Ser Lys Asn
                85                  90                  95

Thr Leu Tyr Leu Gln Met Asn Ser Leu Arg Ala Glu Asp Thr Ala Val
            100                 105                 110

Tyr Tyr Cys Ala Lys Phe Arg Gln Tyr Ser Gly Gly Phe Asp Tyr Trp
        115                 120                 125

Gly Gln Gly Thr Leu Val Thr Val Ser Ser Ala Ser Thr Lys Gly Pro
    130                 135                 140

Ser Val Phe Pro Leu Ala Pro Ser Ser Lys Ser Thr Ser Gly Gly Thr
145                 150                 155                 160

Ala Ala Leu Gly Cys Leu Val Lys Asp Tyr Phe Pro Glu Pro Val Thr
                165                 170                 175

Val Ser Trp Asn Ser Gly Ala Leu Thr Ser Gly Val His Thr Phe Pro
            180                 185                 190

Ala Val Leu Gln Ser Ser Gly Leu Tyr Ser Leu Ser Ser Val Val Thr
        195                 200                 205

Val Pro Ser Ser Ser Leu Gly Thr Gln Thr Tyr Ile Cys Asn Val Asn
    210                 215                 220

His Lys Pro Ser Asn Thr Lys Val Asp Lys Lys Val Glu Pro Lys Ser
225                 230                 235                 240

Cys Asp Lys Thr His Thr Cys Pro Pro Cys Pro Ala Pro Glu Leu Leu
                245                 250                 255

Gly Gly Pro Ser Val Phe Leu Phe Pro Pro Lys Pro Lys Asp Thr Leu
            260                 265                 270

Met Ile Ser Arg Thr Pro Glu Val Thr Cys Val Val Val Asp Val Ser

        275                 280                 285

His Glu Asp Pro Glu Val Lys Phe Asn Trp Tyr Val Asp Gly Val Glu
    290                 295                 300

Val His Asn Ala Lys Thr Lys Pro Arg Glu Glu Gln Tyr Ala Ser Thr
305                 310                 315                 320

Tyr Arg Val Val Ser Val Leu Thr Val Leu His Gln Asp Trp Leu Asn
                325                 330                 335

Gly Lys Glu Tyr Lys Cys Lys Val Ser Asn Lys Ala Leu Pro Ala Pro
            340                 345                 350

Ile Glu Lys Thr Ile Ser Lys Ala Lys Gly Gln Pro Arg Glu Pro Gln
        355                 360                 365

Val Tyr Thr Leu Pro Pro Ser Arg Asp Glu Leu Thr Lys Asn Gln Val
    370                 375                 380

Ser Leu Thr Cys Leu Val Lys Gly Phe Tyr Pro Ser Asp Ile Ala Val
385                 390                 395                 400

Glu Trp Glu Ser Asn Gly Gln Pro Glu Asn Asn Tyr Lys Thr Thr Pro
                405                 410                 415

Pro Val Leu Asp Ser Asp Gly Ser Phe Phe Leu Tyr Ser Lys Leu Thr
            420                 425                 430

Val Asp Lys Ser Arg Trp Gln Gln Gly Asn Val Phe Ser Cys Ser Val
        435                 440                 445

Met His Glu Ala Leu His Asn His Tyr Thr Gln Lys Ser Leu Ser Leu
    450                 455                 460

Ser Pro Gly Lys
465